# EUROPEAN PATENT APPLICATION

(11) **EP 4 667 447 A1**
(43) Date of publication of application: **24.12.2025**
(21) Application number: 24183590.9
(22) Date of filing: 21.06.2024
(51) Int. Cl.: C07C 1/20, C07C 11/09, C08F 110/10

(54) **PROCESS FOR THE PREPARATION OF POLYISOBUTENE FROM BIO-BASED OLEFINS FROM PRIMARY ALCOHOL**

(71) Applicant: BASF SE, 67056 Ludwigshafen am Rhein (DE)
(72) Inventor: WESP, Svenja, 67056 Ludwigshafen am Rhein (DE); BRYM, Markus, Dr., 67056 Ludwigshafen am Rhein (DE); WETTLING, Thomas, Dr., 67056 Ludwigshafen am Rhein (DE); FELDMANN, Oliver, 67056 Ludwigshafen am Rhein (DE); PROKOP, Christian, 67056 Ludwigshafen am Rhein (DE); BAUER, Marcel, 67056 Ludwigshafen am Rhein (DE)
(74) Representative: BASF IP Association

(57) **Abstract**

The present invention relates to the process for preparing lower olefins from primary alcohols. More particularly the present invention relates to the process of conversion of primary alcohol which has at least one hydrogen atom attached to the carbon atom adjacent to the one bearing the alcohol group to lower olefins wherein the primary alcohols are obtained from renewable sources. Furthermore, the present invention relates to a process for preparing polyisobutene from the thus obtainable lower olefins.

## Description

### Field of invention

The present invention relates to the process for preparing lower olefins from primary alcohols. More particularly the present invention relates to the process of conversion of primary alcohol which has at least one hydrogen atom attached to the carbon atom adjacent to the one bearing the alcohol group to lower olefins wherein the primary alcohols are obtained from renewable sources. Furthermore, the present invention relates to a process for preparing polyisobutene from the thus obtainable lower olefins.

### Background

Hydroformylation reaction or "Oxo-process" involves addition of carbon monoxide and hydrogen (H₂) to an unsaturated carbon-carbon double bond of an unsaturated hydrocarbon to prepare an aldehyde compound. On the contrary, reverse-hydroformylation or retro-hydroformylation or dehydroformylation reaction involves conversion of an aldehyde into a corresponding olefin by eliminating syngas (carbon monoxide and dihydrogen). For sustainable development of chemical industry, it is important to develop sustainable and efficient processes for converting renewable biomass derivatives into value-adding chemicals like monomers and polymers thereof. This includes catalytic conversion of a large feedstock of renewable oxygenated biomass derivatives into valuable bulk monomers such as olefins. There is, therefore, a need for continued development of new and sustainable catalytic processes for conversion of renewable oxygenated biomass derivatives such as alcohols and/or aldehydes into valuable bulk monomers such as olefins.

It is well known that catalytic processes for conversion of an alcohol into an olefin involves dehydration reactions (β - elimination reaction). However, alcohol and olefin have same number of carbon atoms.

Processes for conversion of aldehydes into olefins are known in the art.

Kusumoto S., et al (Angew. Chem. Int. Ed. 2015, 54, 8458-8461) discloses retro-hydroformylation reaction of an aldehyde into an alkene and synthesis gas (a mixture of carbon monoxide and dihydrogen) in the presence of a cyclopentadienyl iridium catalyst.

However, the known processes disclose preparing an olefin from an aldehyde by retro-hydroformylation wherein the aldehyde comprises one carbon atom more than the corresponding olefin. Therefore, these processes require extra steps for preparing an olefin from an alcohol, thereby making them cumbersome and expensive.

Biomass is considered as a CO₂ neutral energy carrier and is one of the most abundant and renewable of natural resources. In recent years, both as a result of market conditions as well as in response to a variety of governmental initiatives and mandates, biomass transformation to produce biofuel is has attracted significant effort and investment. With the increased availability and reduced cost of bioethanol, opportunities have been explored to use bioethanol not just as a biofuel but as a feedstock for making a variety of renewable source-derived chemicals.

The most commercially advanced initiative has been the dehydration of bioethanol to produce ethylene, though the conversions of bioethanol to propylene and isobutylene have also been studied. Propylene is an important industrial intermediate for the production of propylene oxide and polypropylene, while isobutylene is similarly widely used for the production of a variety of industrially important products, such as butyl rubber. Both of propylene and isobutylene are obtained through the catalytic or steam cracking of fossil feedstocks, and the development of a commercially viable process for the direct conversion of alcohol to either material would accordingly be of great interest as fossil resources are depleted and/or become more costly to use, especially in consideration of increased demand for both of propylene and isobutylene.

Natural raw materials are in particular substances which are obtained by processing from plants or parts of plants (or even animals). Characteristic of raw materials from renewable sources is a significantly high proportion of the carbon isotope ¹⁴ C. By its determination, the proportion of renewable raw materials can be determined experimentally. Renewable resources differ from those obtained through chemical synthesis or petroleum processing in that they are less homogeneous. The composition of the renewable resources varies considerably as the composition of natural resources depend on factors such as climate and region in which the plant grows, season of harvest, variations between biological species and subspecies and the type of extraction process used in extraction (extrusion, centrifugation, filtration, distillation, cutting, pressing, etc.).

Fusel oils are formed as a by-product of alcoholic fermentation and consist of a mixture of several alcohols comprised mainly of amyl alcohols along with lesser amounts of propanol, n-butanol, and iso-butanol depending upon the purification process employed. Depending on the carbohydrate source for the fermentation process, and the organism used, fusel oil levels are typically between 0.2- 3.0% as a relative percent of the target alcohol produced. Fusel oils are co-products of alcohol fermentation. These oils, which are produced by yeast in anaerobiosis from nitrogenous materials, are recovered after rectification or on the middle plates of a column.

Fusel oils, occasionally referred to as "amyl oils" or "fusels", have compositions which vary depending on their origin (potato, beet, wheat, barley, etc.).They are a mixtures of 5% to 20% of water, 60% to 95% of alcohols mainly consisting of linear or branched alkanols containing from 2 to 5 carbon atoms, of impurities (furfurols, ethers, fatty acids, etc.) which, in extreme cases, may be up to 15% Ethanol, 2-methyl 1-butanol and 3-methyl 1-butanol. Ethanol, 2-methyl 1-butanol and 3-methyl 1- butanol could be used as starting material for the preparation of several olefins which find application in various industries.

Some of the olefins (like Isobutylene, isoamylene) are most commonly used as a starting material for other products as opposed to being used as is for some final applications. While not an exhaustive one, the literature reveals several uses of olefins isobutylene and/or isoamylene. These include (i) hydrocarbon resin modification (softening point/Tg/molecular weight control), (ii) fuel additives via oligomerization (typically dimerization) for octane boosters or via etherification with methanol or ethanol, (iii) synthetic building block such as precursor to diolefins, flavor/fragrance enhancers, antioxidants, typically alkyl phenols, or as synthon for fine chemicals or pharmaceutical ingredients preparation.

Both, homopolymers of isobutene as well as copolymers of isobutene with with olefinically unsaturated monomers copolymerizable with isobutene are referred to as polyisobutene.

Depending on the molecular weight and on the content of terminal double bond the polyisobutene is referred to as
- High molecular polyisobutene with a number-average molecular weight Mₙ of more than 100000 g/mol
- Medium molecular polyisobutene with a number-average molecular weight Mₙ of more than 10000 and up to 100000 g/mol
- Low molecular polyisobutene with a number-average molecular weight Mₙ of more than 350 and up to 10000 g/mol
- Low molecular polyisobutene is usually referred to as highly reactive with a content of terminal (alpha-) double bonds of at least 50 mol%, preferably at least 60, more preferably at least 70, even more preferably at least 80, and especially at least 85 mol%. The content of such alpha-double bonds may be up to 100, up to 98, up to 97 or up to 95 mol%
- Medium molecular polyisobutene is usually referred to as highly reactive with a content of terminal (alpha-) double bonds of 10 to 60 mol%, preferably 15 to 55 mol%, more preferably 20 to 50 mol%, even more preferably 25 to 50 mol%, and especially at least 25 to 45 mol%.

Polyisobutene serves as a valuable starting material for further derivatisation, such as ene reaction with maleic anhydride, epoxidation or hydroformylation which in turn serve as starting material for further reaction, e.g. reaction of polyisobutenyl succinic anhydride with amines, or in the case of hydroformylated polyisobutene subsequent hydrogenation or amination to polyisobutene amine. These products find use e.g. as fuel additives.

Isobutene homopolymers are understood in the context of the present invention to mean those polymers which, based on the polymer, are formed from isobutene to an extent of at least 98 mol%, preferably to an extent of at least 99 mol%. Accordingly, isobutene copolymers are understood to mean those polymers which comprise more than 2 mol% of copolymerized monomers other than isobutene, for example isoprene or linear butenes, preferably 1-butene, cis-2-butene, and trans-2-butene.

Butyl rubbers also are copolymers of isobutene-containing monomer mixtures, however, polyisobutenes usually differ from butyl rubbers in terms of their content of isoprene in the polymer. Polyisobutene copolymers are understood to mean polymers comprising not more than 0.5 mol% isoprene, preferably not more than 0.45 mol%, even more preferably not more than 0.4, and especially not more than 0.35 mol% isoprene and not more than 10 mol%, more preferably not more 5 mol% linear butenes as copolymerized monomers, preferably 1-butene, cis-2-butene, and trans-2-butene.

On industrial scale polyisobutene is usually made from C₄ cuts from steam crackers and are, thus, fossil-based. In order to supersede fossil raw materials it is a subject matter of the present invention to develop a non-fossil source for isobutene.

Thus, an object of the present invention, was to produce olefins from alcohols which are found in renewable feedstock and to convert the thus obtained olefins into polyisobutene.

The further object of the present invention was to produce olefins having pMC greater than 90 using renewable raw materials which are comparable in quality and yield when produced using pure raw materials for the production of polyisobutene.

Yet another object of the present invention, was to develop a robust process which would yield olefins of high quality, higher yield and having pMC greater than 90 irrespective of the nature of the raw materials used (renewable source with several impurities or pure source) for the production of polyisobutene.

Yet another object of the present invention, was to develop a process for the production of polyisobutene in which at least a part of the olefins are obtained from alcohols which are found in renewable feedstock.

### BRIEF DESCRIPTION OF THE DRAWINGS

FIG. 1, graphically illustrates conversion of isoamyl alcohol vs temperature using 0.25 ml of 1.0 wt% Pt / 0.5 wt% Sn / ZrO₂ as catalyst, as represented in Table 2.
FIG. 2, graphically illustrates conversion of isoamyl alcohol vs temperature using 1.0 ml of 1.0 wt% Pt / 0.5 wt% Sn / ZrO₂ as catalyst, as represented in Table 3.
FIG. 3, graphically illustrates conversion of isoamyl alcohol vs temperature using 1.0 ml of 1.0 wt% Pt / 0.5 wt% Sn / ZrO₂ as catalyst at a constant GHSV of 2500 h⁻¹, as represented in Table 4.
FIG. 4, graphically illustrates conversion of isoamyl alcohol vs temperature using 0.25 ml of 1.0 wt% Pt / 0.5 wt% Sn / ZrO₂ as catalyst at a constant GHSV of 9800 h⁻¹, as represented in Table 5.
FIG. 5, graphically illustrates conversion of isoamyl alcohol vs % of isoamyl alcohol using 1.0 ml of 1.0 wt% Pt / 0.5 wt% Sn / ZrO₂ as catalyst, at various GHSV's, at Temp = 300 °C, and Pressure = 1bar, as represented in Table 6.
FIG. 6, graphically illustratess conversion of isoamyl alcohol vs temperature using 0.25 ml of Nickel (60 wt% metal content, Total Pore Volume 0.45 cm³/g) as catalyst, as represented in Table 7.
FIG. 7, graphically illustrates conversion of isoamyl alcohol vs temperature using 1.0 ml of Nickel (60 wt% metal content, Total Pore Volume 0.45 cm³/g) as catalyst, as represented in Table 8.
FIG. 8, graphically illustrates conversion of isoamyl alcohol vs temperature using 1.0 ml of Nickel (56 wt% metal content, Total Pore Volume 0.3 cm³/g) as catalyst, as represented in Table 9.
FIG. 9, graphically illustrates conversion of isoamyl alcohol vs temperature using 1.0 ml Nickel-Copper as catalyst, as represented in Table 10.
FIG. 10, graphically illustrates conversion of isoamyl alcohol vs temperature using 0.25 ml of Nickel-Copper as catalyst, as represented in Table 11.
FIG. 11, graphically illustrates conversion of isoamyl alcohol vs temperature using 1.0 ml of Nickel-Copper as catalyst, as represented in Table 12.
FIG. 12, graphically illustrates conversion of isoamyl alcohol vs temperature using 1.0 ml Nickel-Copper as catalyst, as represented in Table 13.
FIG. 13, graphically illustrates conversion of isoamyl alcohol vs temperature using 0.5 ml of 1.0 wt% Pt / 0.5 wt% Sn / ZrO₂+ Cu-Zn as catalyst, as represented in Table 14.
FIG. 14, graphically illustratess conversion of isoamyl alcohol vs temperature using 0.5 ml of 1.0 wt% Pt / 0.5 wt% Sn / ZrO₂ + Cu-Zn as catalyst, as represented in Table 15.
FIG. 15, graphically illustrates conversion of isoamyl alcohol vs temperature using 1.0 ml of Ru as catalyst, as represented in Table 16.
FIG. 16, graphically illustrates conversion of isoamyl alcohol vs temperature using 1.0 ml of 0.3 % Pd as catalyst, as represented in Table 17.
FIG. 17, graphically illustrates conversion of isoamyl alcohol vs temperature using 1.0 ml of 0.5 % Pd as catalyst, as represented in Table 18.
FIG. 18, graphically illustrates conversion of isoamyl alcohol vs temperature using 1.0 ml of (Pd) as catalyst, as represented in Table 19.

### Summary of the invention

Surprisingly the above objects have been achieved by the process of the present invention.

Thus, the first aspect of the present invention is directed to a process for preparing polyisobutene from a mixture of lower olefins with 4 to 7 carbon atoms, comprising the steps of:
a) feeding into a reaction vessel a stream comprising linear and/or branched C₅-C₈ primary alcohol which has at least one hydrogen atom attached to the carbon atom adjacent to the one bearing the alcohol group,
b) providing a catalyst comprising at least one catalytically active metal selected from platinum (Pt), palladium (Pd), nickel (Ni), Chromium (Cr), Molybdenum (Mo), Ruthenium (Ru), or combinations thereof, and
c) subjecting the primary alcohol to a de-hydroformulation in the presence of the catalyst of step b) yielding an olefin having one carbon atom less than the starting alcohol
d) optionally purifying the reaction product from step c) yielding an olefin-containing product stream, and
e) polymerising the olefin obtained in step c) or d) (if applicable) optionally together with olefin from other sources in the presence of at least one Lewis Acid and in the presence of at least one initiator,
   wherein the linear and/or branched C₅-C₈ primary alcohol comprises 3-Methyl 1-butanol and the olefin comprises isobutene.

According to the invention the starting material in step a) may be mixtures of linear and/or branched C₅-C₈ primary alcohols with the proviso hat such mixtures comprise comprises 3-Methyl 1-butanol. The process according to the invention yields mixtures of the corresponding olefins having one carbon atom less, in this case a mixture of lower olefins with 4 to 7 carbon atoms.

Preferably the starting material may be mixtures of linear and/or branched C₅-C₇ primary alcohols, more preferably mixtures of linear and/or branched C₅-C₆ primary alcohols, even more preferably mixtures of linear and/or branched C₅ primary alcohols, and especially 3-Methyl 1-butanol.

The mixture of olefins obtained from step c) may be purified in step d), preferably by rectification, or may be used in the polymerisation e) as such.

The second aspect of the present invention is directed to a process where the starting material namely the C₅-C₈ primary alcohol has a pMC greater than 90 when measured by a method as described in the ASTM norm D6866 (the current version is D6866-22), which defines the concept of "percent Modern Carbon" or pMC. A "bio-based" material has a pMC of 100%.

The third aspect of the present invention is directed to a process, wherein the starting material namely the linear and/or branched C₅-C₈ primary alcohol which has at least one hydrogen atom attached to the carbon atom adjacent to the one bearing the alcohol group, is converted to at least one lower hydrocarbon having C₄-C₇ carbon atoms at a yield of at least about 80 wt.% of the maximum theoretical molar yield.

Another aspect of the present invention is a process for preparing polyisobutene by polymerising an isobutene-containing olefin mixture in the presence of at least one Lewis Acid and in the presence of at least one initiator, wherein the isobutene in the olefin mixture is at least partly obtained by a process for preparing a lower olefin with 4 to 7 carbon atoms, comprising the steps of:
a) feeding into a reaction vessel a stream comprising linear and/or branched C₅-C₈ primary alcohol which has at least one hydrogen atom attached to the carbon atom adjacent to the one bearing the alcohol group,
b) providing a catalyst comprising at least one catalytically active metal selected from platinum (Pt), palladium (Pd), nickel (Ni), Chromium (Cr), Molybdenum (Mo), Ruthenium (Ru), or combinations thereof, and
c) oxidative decarboxylation of the carboxylic acid to an olefin having one carbon atom less than the starting alcohol,
wherein the linear and/or branched C₅-C₈ primary alcohol comprises 3-Methyl 1-butanol.

Another aspect of the present invention is directed to a process for preparing isobutene starting from 3-methyl-1-butanol obtained from fusel oils using the de-hydroformulation in the presence of the catalyst of step b) to isobutene.

Although the individual steps are known reaction, they have not been described for this particular substrate, and they have not been combined as a means of obtaining isobutene with a pMC greater than 90 (pMC> 90%).

### Description of the invention

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are inter-changeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, min, hours, days, weeks, months or even years between such steps, unless otherwise indicated in the application as set forth herein above or below.

Furthermore, the ranges defined throughout the specification include the end values as well, i.e. a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment but may refer to the same embodiment. Further, as used in the following, the terms "preferably", "more preferably", "even more preferably", "most preferably" and "in particular" or similar terms are used in conjunction with optional features, without restricting alternative possibilities. Thus, features introduced by these terms are optional features and are not intended to restrict the scope of the claims in any way.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any one of the claimed embodiments can be used in any combination.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

The term "yield" is defined as the amount of product obtained per unit weight of raw material and may be expressed as g product /g substrate. Yield may be expressed as a percentage of the theoretical yield. "Theoretical yield" is defined as the maximum amount of product that can be generated per a given amount of substrate as dictated by the stoichiometry of the metabolic pathway used to make the product. For example, if the theoretical yield for one typical conversion of glucose to isobutanol is 0.41 g/g, the yield of butanol from glucose of 0.39 g/g would be expressed as 95% of theoretical or 95% theoretical yield.

The term "biofuel precursor" refers to an organic molecule in which all of the carbon contained within the molecule is derived from biomass and is thermochemically or biochemically converted from a feedstock into the precursor. A biofuel precursor may be a biofuel in its own right or may be configured for conversion, either chemically or biochemically, into a biofuel with different properties. Biofuel precursors include, but are not limited to, 1-propanol, 2-propanol, 1-butanol, 2-butanol, isobutanol, 1-pentanol, isopentanol (3-methyl-1-butanol), 3-pentanol, 2-methyl-1-butanol, or neopentanol.

The terms "alkene" and "olefin" are used interchangeably herein to refer to non-aromatic hydrocarbons having at least one carbon-carbon double bond.

"Carbon of atmospheric origin" as used herein refers to carbon atoms from carbon dioxide molecules that have recently (e.g., in the last few decades) been free in the earth's atmosphere. Such carbon atoms are identifiable by the ratio of particular radioisotopes as described herein. "Green carbon", "atmospheric carbon", "environmentally friendly carbon", "life-cycle carbon", "non-fossil fuel based carbon", "non-petroleum based carbon", "carbon of atmospheric origin", and "biobased carbon" are used synonymously herein.

"Carbon of fossil origin" as used herein refers to carbon of petrochemical origin. Carbon of fossil origin is identifiable by means described herein. "Fossil fuel carbon", "fossil carbon", "polluting carbon", "petrochemical carbon", "petrocarbon" and "carbon of fossil origin" are used synonymously herein.

The term "ASTM" refers to the American Society of Testing and Materials, which defines testing procedures and specifications for all petroleum products manufactured and sold commercially.

Fusel oil as used herein refers to the products that are formed as a by-product of alcoholic fermentation, and consist of a mixture of several alcohols comprised mainly of amyl alcohols along with lesser amounts of propanol, n-butanol, and isobutanol depending upon the purification process employed.

"Renewably-based" or "renewable" denote that the carbon content of the biofuel precursor and subsequent products is from a "new carbon" source as measured by ASTM test method D 6866-05, "Determining the Biobased Content of Natural Range Materials Using Radiocarbon and Isotope Ratio Mass Spectrometry Analysis", incorporated herein by reference in its entirety.

### Process

The present invention is directed to a process for preparing polyisobutene from a lower olefin with 4 to 7 carbon atoms, comprising the steps of:
a) feeding into a reaction vessel a stream comprising linear and/or branched C₅-C₈ primary alcohol which has at least one hydrogen atom attached to the carbon atom adjacent to the one bearing the alcohol group,
b) providing a catalyst comprising at least one catalytically active metal selected from platinum (Pt), palladium (Pd), nickel (Ni), Chromium (Cr), Molybdenum (Mo), Ruthenium (Ru), or combinations thereof, and
c) subjecting the primary alcohol to a de-hydroformulation in the presence of the catalyst of step b) yielding an olefin having one carbon atom less than the starting alcohol
d) optionally purifying the reaction product from step c) yielding an olefin-containing product stream, and
e) polymerising the olefin obtained in step c) or d) (if applicable) optionally together with olefin from other sources in the presence of at least one Lewis Acid and in the presence of at least one initiator,
wherein the linear and/or branched C₅-C₈ primary alcohol comprises 3-Methyl 1-butanol and the olefin comprises isobutene.

### Step a)

In step (a), the linear and/or branched C₅-C₈ primary alcohol has at least one hydrogen atom attached to the carbon atom adjacent to the one bearing the alcohol group.

The linear and/or branched C₅-C₈ primary alcohol has at least one hydrogen atom attached to the carbon atom adjacent to the one bearing the alcohol group that is the alpha carbon atom.

In an embodiment, the linear and/or branched C₅-C₈ primary alcohol has at least one hydrogen atom attached to the carbon atom adjacent to the one bearing the alcohol group that is the alpha carbon atom and the beta carbon atom 9 (i.e the carbon atom attached to the first carbon atom adjacent to the one bearing the alcohol group) also has at least one hydrogen atom.

In a further embodiment, the C₅-C₈ primary alcohol is obtained from natural sources or obtained from fermentation process.

In an embodiment the C₅-C₈ primary alcohol is obtained from fusel oil.

### Fusel oil

Fusel oil is well known in the art and comprises a mixture of light alcohols, fatty esters, terpenes and furfural. The alcohols comprised in fusel oil are mainly propanol, butanol, amyl alcohol, isoamyl alcohols and hexanol and optionally heavier linear alcohols such as C₇ or C₈ alcohols.

Fusel oils, occasionally referred to as "amyl oils" or "fusels", have compositions which vary depending on their origin (potato, beet, wheat, barley, etc. musts).

Fusel oils form colourless or yellowish liquids, which have a characteristic odour. They have a density of about 0.83. Their boiling point is far from constant, since they are complex mixtures of substances with a very variable boiling point. Boiling commences at about 80° C. and rises to 130-134°C. They are insoluble in water and are usually washed with water and separated out by settling of the phases in order to reduce the amount of ethanol they contain by about 4% to 5%. It should be noted that fusel alcohols are natural alcohols directly produced via biotechnology in distilleries, without any intermediate chemical step.

Fusel oil may be obtained by several processes well known from the skilled person, e.g. by direct removal in the distillation column and cooling. The removed fraction can be purified e.g. by extraction and decantation. A liquid/liquid extraction by addition of water followed by a decantation leads to the formation of two phases. The upper phase comprises mainly amyl and butyl alcohols, slightly soluble in water. The various fractions of fusel oil may also be separated by using adsorbents, which are regenerated thereafter. Among the tested adsorbents, granulated vegetal activated charcoal is preferred since it is able to adsorb eight times its weight of fusel oil.

In an embodiment the fusel oil contains a mixture of linear or branched C₅ alcohols, C₄ alcohols or C₃ alcohols.

According to a preferred embodiment, C₅ branched alcohol present in the initial composition is a mixture of isoamyl alcohol and amyl alcohol, i.e. 3-methylbutan-1-ol (isoamyl alcohol) and 2-methylbutan-1-ol (amyl alcohol).

According to a preferred embodiment, the initial composition comprises at least 30 wt%, preferably at least 40 wt%, more preferably at least 50 wt%, more preferably at least 60 wt %, even more preferably at least 70 wt% C₅ branched alcohols, based on the total weight of the composition.

C₄ alcohols may also be present in the initial composition, for example, butan-1-ol and 2-methylpropan-1-ol. The initial composition may comprise one of these C₄ alcohols or both.

C₃ alcohols may also be present in the initial composition, for example, n-propanol. The initial composition may comprise 0.01 to 20 wt% of C₃ alcohol.

Fusel oil may further contain hexanol and optionally heavier linear alcohols such as C₇ or C₈ alcohols.

Fusel oil is a mixture of 5% to 20% of water, 60% to 95% of alcohols mainly consisting of linear or branched alkanols containing from 2 to 5 carbon atoms of impurities including but not limiting to furfurols, ethers, fatty acids, etc. which, may be up to 15%.

In an embodiment the composition of fusel oil is as follows:
- Ethanol 5 to 40%,
- 1-Propanol 1 to 8%,
- 2-Propanol 0 to 1%,
- 2-Methylpropanol 5 to 15%,
- 1-Butanol-0 to 1%,
- 2-Methyl 1- butanol 10 to 30%,
- 3-Methyl 1-butanol (isoamyl alcohol)25 to 70%,
the combination of alkanols representing 100%.

In an embodiment, the C₅-C₈ primary alcohol is selected from 2-methyl-1-butanol (active amyl alcohol), 3-Methyl 1-butanol(isoamyl alcohol, isopentanol), n-pentanol, n-hexanol, 2-methylpentanol, n-heptanol, n-octanol, 2-ethylhexanol or mixtures thereof.

In a preferred embodiment, the C₅-C₈ primary alcohol is 3-Methyl 1-butanol (isoamyl alcohol).

In an embodiment, the stream which is obtained from the fusel oil contains the C₅-C₈ primary alcohol at a concentration in the range of 60-99wt % preferably in the range of 60-95wt %, preferably in the range of 60-90wt %, preferably in the range of 60-85wt %, preferably in the range of 60-80wt %, more preferably in the range of 65-80wt %, even more preferably in the range of 70-80wt %.

In an embodiment, the stream which is obtained from the fusel oil contains the C₅-C₈ primary alcohol has a pMC greater than 90 when measured by a method as described in the ASTM norm D6866 (the current version is D6866-22), which defines the concept of "percent Modern Carbon" or pMC. Preferably the pMC is greater than 91, preferably greater than 93, preferably greater than 95, preferably greater than 96, preferably greater than 97, more preferably greater than 98, even more preferably greater than 99.

In an embodiment, the verification that a feedstock was derived from renewable raw materials is possible according to ASTM D6866 via ¹⁴C for example. A feedstock shall be regarded as "derived from renewable raw materials" for the purposes of this invention when the carbon-14 (C-14) presence therein corresponds substantially (to within not more than 6%) to the ASTM D6866 content of C-14 in atmospheric CO₂.

The C-14 content of a material may be determined by determining the decays of C-14 in this material by liquid scintillation. Such raw materials shall preferably be regarded as derived from renewable raw materials when they have a C-14 content displaying a radioactive decay of not less than 1.5 dpm/gC (decays per minute per gram of carbon), preferably 2 dpm/gC, more preferably 2.5 dpm/gC and yet more preferably 5 dpm/gC.

"Renewably-based" or "renewable" denote that the carbon content of the biofuel precursor and subsequent products is from a "new carbon" source as measured by ASTM test method D 6866-05, "Determining the Biobased Content of Natural Range Materials Using Radiocarbon and Isotope Ratio Mass Spectrometry Analysis", incorporated herein by reference in its entirety. This test method measures the ¹⁴C/¹²C isotope ratio in a sample and compares it to the ¹⁴C/¹²C isotope ratio in a standard 100% biobased material to give percent biobased content of the sample. "Biobased materials" are organic materials in which the carbon comes from recently (on a human time scale) fixated CO₂ present in the atmosphere using sunlight energy (photosynthesis). On land, this CO₂ is captured or fixated by plant life (e.g., agricultural crops or forestry materials). In the oceans, the CO₂ is captured or fixated by photosynthesizing bacteria or phytoplankton. For example, a biobased material has a ¹⁴C/¹²C isotope ratio greater than 0. Contrarily, a fossil-based material, has a ¹⁴C/¹²C isotope ratio of about 0. The term "renewable" with regard to compounds such as alcohols or hydrocarbons (linear or cyclic alkanes/alkenes/alkynes, aromatic, etc.) refers to compounds prepared from biomass using thermochemical methods (e.g., Fischer-Tropsch catalysts), biocatalysts (e.g., fermentation), or other processes, for example as described herein.

A small amount of the carbon atoms of the carbon dioxide in the atmosphere is the radioactive isotope ¹⁴C. This ¹⁴C carbon dioxide is created when atmospheric nitrogen is struck by a cosmic ray generated neutron, causing the nitrogen to lose a proton and form carbon of atomic mass 14 (¹⁴C), which is then immediately oxidized to carbon dioxide. A small but measurable fraction of atmospheric carbon is present in the faun of 14CO₂. Atmospheric carbon dioxide is processed by green plants to make organic molecules during the process known as photosynthesis. Virtually all forms of life on Earth depend on this green plant production of organic molecule to produce the chemical energy that facilitates growth and reproduction. Therefore, the ¹⁴C that forms in the atmosphere eventually becomes part of all life forms and their biological products, enriching biomass and organisms which feed on biomass with ¹⁴C. In contrast, carbon from fossil fuels does not have the signature 14C:12C ratio of renewable organic molecules derived from atmospheric carbon dioxide. Furthermore, renewable organic molecules that biodegrade to CO₂ do not contribute to global warming as there is no net increase of carbon emitted to the atmosphere.

Assessment of the renewably based carbon content of a material can be performed through standard test methods, e.g. using radiocarbon and isotope ratio mass spectrometry analysis. ASTM International (formally known as the American Society for Testing and Materials) has established a standard method for assessing the biobased content of materials. The ASTM method is designated ASTM-D6866.

The application of ASTM-D6866 to derive "biobased content" is built on the same concepts as radiocarbon dating, but without use of the age equations. The analysis is performed by deriving a ratio of the amount of radiocarbon (¹⁴C) in an unknown sample compared to that of a modern reference standard. This ratio is reported as a percentage with the units "pMC" (percent modern carbon). If the material being analyzed is a mixture of present day radiocarbon and fossil carbon (containing very low levels of radiocarbon), then the pMC value obtained correlates directly to the amount of biomass material present in the sample.

The C₅-C₈ primary alcohols used in the present invention have pMC values of at least greater than 90, preferably pMC values of at least greater than 95, preferably pMC values of at least greater than 98, more preferably pMC values of at least greater than 99, more preferably pMC values of at least about 100, inclusive of all values and subranges there- between.

### Step b)

In step b) catalyst a catalyst is provided comprising at least one catalytically active metal selected from platinum (Pt), palladium (Pd), nickel (Ni), Chromium (Cr), Molybdenum (Mo), Ruthenium (Ru), or combinations thereof, preferably at least one catalytically active metal selected from Platinum (Pt), Palladium (Pd), Nickel (Ni), or combinations thereof

In a preferred embodiment the catalyst may further comprise at least one promoter selected from Tin (Sn), Silver (Ag), Iridium (Ir), Rhenium (Re), or combinations thereof, wherein the at least one promoter is present in an amount in the range from 0.005 wt% to 5.0 wt%, with respect to total weight of the catalyst. Preferably the at least one promoter is selected from Tin (Sn) and Rhenium (Re).

In a preferred embodiment, the at least one promoter is present in an amount in the range from 0.1 wt% to 3.0 wt%, with respect to total weight of the catalyst, preferably in the range from 0.2 wt% to 2.0 wt%, more preferably in the range from 0.3 wt% to 1.5 wt%, and especially in an amount of 0.5 wt%, with respect to total weight of the catalyst.

In a preferred embodiment, in the catalyst the at least one catalytically active metal may be supported on at least one carrier on which the catalytically active metal is supported, wherein the carrier is selected from a non-acidic material selected from calcium oxide, titanium oxide, beryllia, magnesia, thoria, zirconia, alumina, silicon carbide, quartz or combinations thereof,
wherein the at least one carrier is present in an amount in the range from 90.0 wt% to 99.995 wt%, with respect to total weight of the catalyst.

Preferably the carrier is selected from a non-acidic material selected from titanium oxide, zirconia alumina, or combinations thereof.

In a preferred embodiment, the at least one catalyst comprises
a. at least one catalytically active metal selected from Platinum (Pt), Palladium (Pd), Nickel (Ni), Chromium (Cr), Molybdenum (Mo), Ruthenium (Ru), or combinations thereof;
b. at least one promoter selected from Tin (Sn), Silver (Ag), Iridium (Ir), Rhenium (Re), or combinations thereof; and
c. at least one carrier on which the catalytically active metal is supported, wherein the carrier is selected from a non-acidic material selected from calcium oxide, titanium oxide, beryllia, magnesia, thoria, zirconia, alumina, silicon carbide, quartz or combinations thereof.

In a preferred embodiment, the at least one catalyst comprises at least one catalytically active metal Platinum (Pt), at least one promoter Tin (Sn), and at least one carrier Zirconia (ZrO₂).

In the present invention the crystalline phase of the carrier is stable under the conditions of the reaction involving conversion of an alcohol into an aldehyde followed by reverse-hydroformylation or retro-hydroformylation or dehydroformylation reaction involving conversion of an aldehyde into a corresponding olefin by eliminating syngas (carbon monoxide and dihydrogen).

The preparation of the at least one catalyst comprising at least one catalytically active metal Platinum (Pt), at least one promoter Tin (Sn), and at least one carrier Zirconia (ZrO₂) of the present invention, as disclosed in US patent No. 6,989,346 B2 is specifically incorporated by reference herein.

In another preferred embodiment, the at least one catalyst further comprises other possible elements that are known to influence the acidity of the catalyst surface and/or to stabilize the catalytically active metals against sintering. For example, elements of main groups I and II, i.e. Li, Na, K, Rb, Cs on the one hand and Mg, Ca, Sr and Ba on the other hand, elements of main group III i.e. gallium, indium and thallium, and elements of transition group III i.e. Y and La and also rare earth elements. When tetragonal ZrO₂ is employed as a carrier, it can be stabilized by doping with Lanthanum (La) or Yttrium (Y). Zinc has also been found to be effective.

In another preferred embodiment the at least one catalyst has a BET surface area in the range from 1 to 500 square meters per gram (m²/g), as measured by the Brunauer-Emmett-Teller method, preferably from 5 to 300 square meters per gram (m²/g), even more preferably from 10 to 200 square meters per gram (m²/g), and especially from 20 to 100 square meters per gram (m²/g), as measured by the Brunauer-Emmett-Teller method.

In the presently claimed invention, the amount of the at least one catalytically active metal that can be effectively disposed upon a suitable carrier varies depending usually upon the surface area of the carrier.

The amount of the at least one promoter present in the catalyst varies depending upon the at least one catalytically active metal and the hydrocarbon feed employed.

Promoter has been found to help inhibit isomerization and cracking of hydrocarbon feed during the high temperature catalytic processes involving precious metals such as Platinum and Palladium. Promoter has also been found to stabilize the catalyst and extend its life. The catalyst can be regenerated by conventional oxidation of the catalyst.

In a preferred embodiment, the at least one carrier support is non-acidic refractory material selected from calcium oxide, titanium oxide, beryllia, magnesia, thoria, zirconia, alumina, silica, silicon carbide, quartz or combinations thereof.

Preferably, the at least one carrier support is non-acidic refractory material selected from calcium oxide, titanium oxide, magnesia, zirconia, alumina, silica, or combinations thereof.

More preferably, the at least one carrier support is non-acidic refractory material selected from magnesia, zirconia, titanium oxide, alumina, silica, or combinations thereof.

More preferably, the at least one carrier support is non-acidic refractory material zirconia.

In a preferred embodiment, the at least one carrier is present in an amount in the range from 95.0 wt% to 99.5 wt%, with respect to total weight of the catalyst.

Preferably, the at least one carrier is present in an amount in the range from 97.0 wt% to 99.0 wt%, with respect to total weight of the catalyst.

Preferably, the at least one carrier is present in an amount of 98.5 wt%, with respect to total weight of the catalyst.

In a preferred embodiment, the at least one catalyst has a pore volume in the range from 0.1 to 1.0 ml/g, as determined by Mercury (Hg) Porosimetry, preferably in the range from 0.15 to 0.6 ml/g, more preferably in the range from 0.2 to 0.4 ml/g, as determined by Mercury (Hg) Porosimetry.

In a preferred embodiment, the at least one catalyst has a mean pore diameter, as determined by Mercury (Hg) Porosimetry, in the range from 0.008 to 0.06 microns (µ), preferably in the range from 0.01 to 0.04 microns (µ).

In a preferred embodiment, the at least one catalytically active metal comprises Nickel (Ni). Preferably, the catalyst has a Nickel content in the range from 10 wt% to 70 wt%, preferably from 50.0 wt% to 65.0 wt.%. In a preferred embodiment, the catalyst having catalytically active metal comprising Nickel (Ni) has a total pore volume in the range from 0.25 cm³/g to 0.5 cm³/g.

The remainder of the catalyst may be an oxidic bonding material, for example aluminium oxide, or zirconium dioxide. Preferably, the Ni-containing catalysts are bulk catalysts or precipitation-type catalysts, as opposed to supported catalysts.

In a preferred embodiment, the at least one catalytically active metal is Nickel (Ni).

In a preferred embodiment, the at least one catalytically active metal is Nickel-Copper (Ni-Cu). Preferably, the molar ratio of nickel to copper is greater than 1, preferably greater than 1.2, more preferably 1.8 to 8.5.

Preferably, the at least one catalytically active metal Nickel (Ni) has Nickel content in the range from 50.0 wt% to 65.0 wt.%; and a total pore volume in the range from 0.25 cm³/g to 0.5 cm³/g.

It is well known that pure nickel catalysts tend to deposit carbon when exposed to an atmosphere containing CO or hydrocarbons (as starting materials or end products). On the other hand copper, irrespective of whether it is active or inactive as a catalyst for that reaction, does not show any tendency to deposit carbon. Therefore, nickel-copper catalysts are used for catalyzing reactions in which carbon monoxide and/or hydrocarbons are present in the reaction gas phase (as starting materials or end products) or are intermediately formed, in order to inhibit carbon deposition on the catalyst. Similarly, copper and copper alloys can be used in combination with other catalysts used for catalyzing reactions in which carbon monoxide and/or hydrocarbons are present in the reaction gas phase or are intermediately formed, in order to inhibit carbon deposition on the catalyst. In addition, presence of copper can strengthen the nickel based catalysts against attrition during catalytic reactions at high temperature and pressure conditions.

In general, precipitation methods are used for the preparation of the Ni-containing catalysts. Thus, they can be obtained, for example, by coprecipitation of the nickel and, optionally, copper components, from an aqueous salt solution containing these components, by means of a base, in the presence of a suspension of a sparingly soluble, oxidic bonding material, and subsequent washing, drying and calcination of the resulting precipitate.

For precipitation, a base, in particular an aquoeous alkali metal base, for example sodium carbonate, sodium hydroxide, potassium carbonate or potassium hydroxide, is added to an aqueous salt solution containing catalyst components at elevated temperatures and with stirring, until the precipitation is complete. The type of salts used is in general not critical and salts having high aqueous solubility are generally preferred.

In a preferred embodiment, the at least one catalytically active metal comprises Nickel (Ni), and the step of contacting is carried out at a temperature in the range from 200 °C to 400 °C, preferably 200 to 300 °C, more preferably 200 to 250 °C. When the at least one catalytically active metal is Nickel-Copper (Ni-Cu), a temperature in the range of from 250 to 350 °C is particularly useful.

In a preferred embodiment, the at least one catalyst has a particle size in the range from 50 µm to 500 µm, preferably from 100 µm to 350 µm.

In a preferred embodiment, the at least one catalyst has a bed volume in the range from 0.1 ml to 1.5 ml, preferably from 0.2 ml to 1.1 ml.

### Step c)

Step c) is a reverse-hydroformylation or retro-hydroformylation or dehydroformylation reaction of the primary alcohol which involves conversion of an aldehyde into the corresponding olefin by eliminating syngas (carbon monoxide and dihydrogen).

Step c) is conducted at a partial pressure in the range of 0.02 to 4.0 bar, and at a temperature in the range of 200 °C to 450 °C, preferably 250 °C to 450 °C, more preferably 300 to 450 °C, even more preferably 300 °C to 400 °C, especially 300 °C to 350 °C.

To keep the catalyst beds on a constant level, the catalyst material can be diluted with inert particles in such way that all reactors had a constant catalyst bed volume.

In a preferred embodiment, the step of contacting involves contacting a feed stream comprising the at least one linear and/or branched C₅-C₈ primary alcohol which has at least one hydrogen atom attached to the carbon atom adjacent to the one bearing the alcohol group in a gas phase.

In a preferred embodiment, the feed stream is fed at a gas hourly space velocity (GHSV) in the range from 100 h⁻¹ to 15000 h⁻¹.

In a preferred embodiment, the feed stream is fed at a gas hourly space velocity (GHSV) in the range from 300 h⁻¹ to 10000 h⁻¹.

In a preferred embodiment, the feed stream is fed at a gas hourly space velocity (GHSV) in the range from 300 h⁻¹ to 3000 h⁻¹.

In a preferred embodiment, amount of the at least one primary alcohol in the feed stream is in the range from 2.0 wt% to 100.0 wt% based on the total weight of the feed stream.

In a preferred embodiment, amount of the at least one primary alcohol in the feed stream is in the range from 75.0 wt% to 95.0 wt% based on the total weight of the feed stream.

In a preferred embodiment, the feed stream further comprises at least one inert gas selected from N₂, CO₂, CH₄ or Ar.

Inert gases such as nitrogen (N₂), Carbon-di-oxide (CO₂), Argon (Ar), or methane (CH₄) can be used as diluent to adjust partial pressure of a hydrocarbon feed.

Hydrogen (H₂) gas when used as a diluent can act as an inhibitor, thereby inhibiting the coke formation on the catalyst and help improve the catalyst performance.

The skilled person will appreciate that the method is carried out in an essentially non-oxidative atmosphere, which means that the feed stream is essentially free of gaseous oxidants such as air, oxygen, ozone, nitrous oxide and nitric oxide. In particular, the feed stream is essentially free of molecular oxygen, for example, the feed stream contains less than 5 vol.-%, more preferably less than 1 vol.-% of molecular oxygen.

In a preferred embodiment, the isobutene is obtained in the form of a product stream.

In a preferred embodiment, the product stream comprises isobutene, optionally other olefins, carbon monoxide and hydrogen.

In a preferred embodiment, the step of contacting further involves a step of separating carbon monoxide and hydrogen from the product stream to obtain the at least one olefin.

### Step d) (optionally)

In optional step d) the reaction product from step c) is purified to yield an olefin-containing product stream which can be further employed in step e).

In a preferred embodiment, step d) is undergone in the course of the process according to the present invention.

The reaction product of step c) usually is a mixture comprising the olefin as target product, optionally olefins other than the target product, the starting alcohol and the aldehyde corresponding to the starting alcohol.

The purification in step d) is preferably selected from the group consisting of
- distillation,
- extraction,
- membrane filtration,
- reverse osmosis, and
- sorption to inorganic materials.

Preferably the purification is conducted by distillation and/or extraction, more preferably by distillation or rectification.

Purification is preferably carried out by distillation or rectification, optionally supported by stripping with an inert gas. This can also be done by increasing the temperature of the reaction mixture and/or lowering the pressure, preferably by a combination of these two measures.

Single-stage distillation can be done either from the reactor or by passing through a suitable aggregate, such as rotary evaporator, thin film evaporator, falling film evaporator, wiper blade evaporator, sambay evaporator, etc., and combinations thereof.

Rectification is preferably carried out by distillation columns placed above the reactor, such as bottom columns, which can be equipped with internals, valves, side extractors, etc. if desired. The distillation column or columns used can be realized in a well-known design (see e.g. Sattler, Thermische Trennverfahren, 2nd edition 1995, Weinheim, pp. 135ff; Perry's Chemical Engineers Handbook, 7th ed. 1997, New York, Section 13). The distillation columns used may contain separating fixtures, such as separating floors, e.g. perforated bottoms, bell bottoms or valve bottoms, orderly packings, e.g. sheet or fabric packings, or irregular pouring of fillers. As a rule, up to 20, preferably up to 10 theoretical plates are sufficient.

An extraction is preferably carried out using polar solvents in order to separate the polar compounds in the reaction mixture from the hydrocarbons.

Suitable extractants may e.g. be n-butanol, iso-butanol, sek-butanol, 2-ethylhexanol, 2-propylheptanol, cyclopentanol, cyclohexanol, the C₅- C₈ primary alcohol serving as starting material, C₂-C₁₀-carboxylic acid C₁-C₁₀-alkyl esters, phthalic acid-C₁-C₁₀-alkyl esters, ketones, such as acetone, ethyl methyl ketone or diethyl ketone.

It is also possible to use hydrocarbons as extractants in order to separate the hydrocarbons in the reaction mixture from the polar compounds.

Such hydrocarbons as extractants are e.g. halogen-free aliphatic or cycloaliphatic, preferably aliphatic hydrocarbons, especially hexane, pentane, heptane, cyclohexane, cyclopentane, and mixtures comprising them. In a preferred embodiment the extractant is the same hydrocarbon which is also used as solvent in the polymerisation (see below).

In terms of process technology, all known extraction and washing processes and apparatus can be used for washing or extraction in step d), e.g. those described in Ullmann's Encyclopedia of Industrial Chemistry, 6th ed, 1999 Electronic Release, Chapter: Liquid - Liquid Extraction - Apparatus. For example, these can be single- or multi-stage, preferably single-stage extractions, as well as those in direct or counter-current mode, preferably counter-current operation.

Preferably, sieve bottom or packed or packing columns, stirring tanks or mixer-settler devices, as well as pulsed columns or those with rotating internals are used.

Membrane filtration and reverse osmosis are known to the person skilled in the art and do not need to be explained further.

Sorption can occur on inorganic materials, such as silica gel, silicates, alumina, zeolites, diatomaceous earth, mixed aluminium/silicon oxides, as well as calcium carbonates and oxides, or on activated or charcoal.

### Step e)

In step e) the olefin obtained in step c) or d) (if applicable) is polymerised in the presence of at least one Lewis Acid and in the presence of at least one initiator.

The olefin obtained in step c) or d) may optionally be mixed with isobutene from other sources, preferably fossil sources. Hence, it is possible to obtain polyisobutene with a certain content of monomers from renewable as well as fossil sources.

In a preferred embodiment, the olefin utilised in step e) predominantly originates from step c) and having a pMC greater than 90, preferably at least 95, more preferably at least 98%, even more preferably at least 99%, or even 100%, when measured by a method as described in the ASTM norm D6866, preferably to an extent of at least 66%, more preferably to an extent of at least 75%, even more preferably to an extent of more than 85%, especially to at least 90%, and even completely from step c).

In case the isobutene completely originates form step c) the resulting polyisobutene exhibits a pMC of at least 90%, preferably at least 95%, more preferably at least 98% , even more preferably at least 99%, or even 100%, when measured by a method as described in the ASTM norm D6866.

It is an advantage of polyisobutene obtained from this preferred embodiment that it is especially suitable as food grade or polyisobutene or for medical applications, e.g. for chewing gums, in adhesive compositions approved for food contact or in plasters.

In another embodiment of the present invention isobutene from step c) or d) from renewable sources may be admixed to isobutene from fossil sources to an extent of of at least 1%, preferably at least 2%, more preferably at least 5% , even more preferably at least 10%, and especially at least 25%. Higher contents are possible depending on the availability of isobutene from renewable sources, especially from the steps a) to c).

For the use of fossil isobutene or of an isobutene-comprising monomer mixture as the monomer to be polymerized, suitable isobutene sources are both pure isobutene and isobutenic C4 hydrocarbon streams, for example C4 raffinates, especially "raffinate 1", C4 cuts from isobutane dehydrogenation, C4 cuts from steam crackers and from FCC crackers (fluid catalyzed cracking), provided that they have been substantially freed of 1,3-butadiene present therein. A C4 hydrocarbon stream from an FCC refinery unit is also known as "b/b" stream. Further suitable isobutenic C4 hydrocarbon streams are, for example, the product stream of a propylene-isobutane cooxidation or the product stream from a metathesis unit, which are generally used after customary purification and/or concentration. Suitable C4 hydrocarbon streams generally comprise less than 500 ppm, preferably less than 200 ppm, of butadiene. The presence of 1-butene and of cis- and trans-2-butene is substantially uncritical. Typically, the isobutene concentration in the C4 hydrocarbon streams mentioned is in the range from 40 to 60% by weight. For instance, raffinate 1 generally consists essentially of 30 to 50% by weight of isobutene, 10 to 50% by weight of 1-butene, 10 to 40% by weight of cis- and trans-2-butene, and 2 to 35% by weight of butanes; in the polymerization process according to the invention, the unbranched butenes in the raffinate 1 generally behave virtually inertly, and only the isobutene is polymerized.

In a preferred embodiment, the fossil monomer source used for the polymerization is a technical C4 hydrocarbon stream with an isobutene content of 1 to 100% by weight, especially of 1 to 99% by weight, in particular of 1 to 90% by weight, more preferably of 30 to 60% by weight, especially a raffinate 1 stream, a b/b stream from an FCC refinery unit, a product stream from a propylene-isobutane cooxidation or a product stream from a metathesis unit.

Especially when a raffinate 1 stream is used as the isobutene source, the use of water as the sole initiator or as a further initiator has been found to be useful, in particular when polymerization is effected at temperatures of - 20°C to +30°C, especially of 0°C to +20°C. At temperatures of -20°C to +30°C, especially of 0°C to +20°C, when a raffinate 1 stream is used as the isobutene source, it is, however, also possible to dispense with the use of an initiator.

The raw material of C4 compounds is usually selected from the group consisting of
(a) a C4 compound material in which an isobutene amount is adjusted to 50 to 75% by weight, obtained by adding high purity isobutene having the isobutene amount of 90 to 100% by weight to C4 raffinate-1 which is a remainder after extracting 1,3-butadiene from a C4 compound derived during a naphtha degrading process;
(b) a C4 compound material in which an isobutene amount is adjusted to 50 to 75% by weight, obtained by adding high amount isobutene mixture having isobutene amount of 80 to 97% by weight, which is generated in an olefin conversion unit (OCU) process that produces propylene by the metathesis of ethylene and 2-butene, to C4 raffinate-1 which is a remainder after extracting 1,3-butadiene from a C4 compound derived during a naphtha degrading process;
(c) a C4 compound material in which an isobutene amount is adjusted to 50 to 75% by weight, obtained by adding high purity isobutene having the isobutene amount of 90 to 100% by weight to butane-butene oil (B-B oil) derived from crude oil refining process;
(d) a C4 compound material in which an isobutene amount is adjusted to 50 to 75% by weight, obtained by adding high amount isobutene mixture having the isobutene amount of 80 to 97% by weight, which is generated in an olefin conversion unit (OCU) process that produces propylene by the metathesis of ethylene and 2-butene, to butane-butene oil (B-B oil) derived from crude oil refining process;
(e) a C4 compound material in which an isobutene amount is adjusted to 50 to 75% by weight, obtained by adding a dilute solvent to high purity isobutene having an isobutene amount of 90 to 100% by weight;
(f) a C4 compound material in which an isobutene amount is adjusted to 50 to 75% by weight, obtained by adding a dilute solvent to high amount isobutene mixture having the isobutene amount of 80 to 97% by weight, which is generated in an olefin conversion unit (OCU) process that produces propylene by the metathesis of ethylene and 2-butene;
(g) a C4 compound material in which an isobutene amount is adjusted to 50 to 75% by weight, obtained by adding high purity isobutene having the isobutene amount of 90 to 100% by weight to a mixture generated in dehydrogenation reaction that converts isobutane to isobutene; and
(h) a C4 compound material in which an isobutene amount is adjusted to 50 to 75% by weight, obtained by adding high amount isobutene mixture having the isobutene amount of 80 to 97% by weight, which is generated in an olefin conversion unit (OCU) process that produces propylene by the metathesis of ethylene and 2-butene to a mixture generated in dehydrogenation reaction that converts isobutane to isobutene.

The isobutenic monomer mixture mentioned may comprise small amounts of contaminants such as water, carboxylic acids or mineral acids, without there being any critical yield or selectivity losses. It is appropriate to prevent enrichment of these impurities by removing such harmful substances from the isobutenic monomer mixture, for example by adsorption on solid adsorbents such as activated carbon, molecular sieves or ion exchangers.

It is also possible to convert monomer mixtures of isobutene or of the isobutenic hydrocarbon mixture with olefinically unsaturated monomers copolymerizable with isobutene. When monomer mixtures of isobutene are to be copolymerized with suitable comonomers, the monomer mixture preferably comprises at least 5% by weight, more preferably at least 10% by weight and especially at least 20% by weight of isobutene, and preferably at most 95% by weight, more preferably at most 90% by weight and especially at most 80% by weight of comonomers.

Useful copolymerizable monomers include: vinylaromatics such as styrene and α-methylstyrene, C1- to C4-alkylstyrenes such as 2-, 3- and 4-methylstyrene, and 4-tert-bu-tylstyrene, halostyrenes such as 2-, 3- or 4-chlorostyrene,
and isoolefins having 5 to 10 carbon atoms, such as 2-methylbutene-1, 2-methylpentene-1, 2-methylhexene-1, 2-ethylpentene-1, 2-ethylhexene-1 and 2-propylheptene-1. Further useful comonomers include olefins which have a silyl group, such as 1-trimethoxysilylethene, 1-(tri-methoxysilyl)propene, 1-(trimethoxysilyl)-2-methylpropene-2, 1-[tri(methoxyethoxy)-silyl]-ethene, 1-[tri(methoxyethoxy)silyl]propene, and 1-[tri(methoxyethoxy)silyl]-2-methylpro-pene-2. In addition - depending on the polymerization conditions - useful comonomers also include isoprene, 1-butene and cis- and trans-2-butene.

When the process according to the invention is to be used to prepare copolymers, the process can be configured so as to preferentially form random polymers or to preferentially form block copolymers. To prepare block copolymers, for example, the different monomers can be supplied successively to the polymerization reaction, in which case the second comonomer is especially not added until the first comonomer is already at least partly polymerized. In this manner, diblock, triblock and higher block copolymers are obtainable, which, according to the sequence of monomer addition, have a block of one or the other comonomer as a terminal block. In some cases, however, block copolymers also form when all comonomers are supplied to the polymerization reaction simultaneously, but one of them polymerizes significantly more rapidly than the other(s). This is the case especially when isobutene and a vinylaromatic compound, especially styrene, are copolymerized in the process according to the invention. This preferably forms block copolymers with a terminal polystyrene block. This is attributable to the fact that the vinylaromatic compound, especially styrene, polymerizes significantly more slowly than isobutene.

The polymerization can be effected either continuously or batchwise.

The process according to the invention is suitable either for performance at low temperatures, e.g. at -90°C to 0°C, or at higher temperatures, i.e. at at least 0°C, e.g. at 0°C to +30°C or at 0°C to +50°C. The polymerization in the process according to the invention is, however, preferably performed at relatively low temperatures, generally at -70°C to -10°C, especially at -60°C to -15°C.

When the polymerization in the process according to the invention is effected at or above the boiling temperature of the monomer or monomer mixture to be polymerized, it is preferably performed in pressure vessels, for example in autoclaves or in pressure reactors.

Step e) comprises polymerizing isobutene or an isobutene-comprising monomer mixture mixture which is at least partly obtained by a process comprising the steps a) to c) in the presence of at least one Lewis Acid effective as a polymerization catalyst.

The Lewis Acid is preferably selected from the group consisting of aluminium trihalide, alkylaluminium halide, iron trihalide, a gallium trihalide, a titanium tetrahalide, a zinc dihalide, a tin dihalide, a tin tetrahalide, and a boron trihalide, more preferably selected from the group consisting of aluminium trihalide, alkylaluminium halide, titanium tetrahalide, and boron trihalide, even more preferably selected from the group consisting of aluminium trihalide, alkylaluminium halide, and boron trihalide, and especially the metal halide is aluminium trihalide or boron trihalide or even aluminium trichloride or boron trifluoride.

In a preferred embodiment, the Lewis Acid is deployed together with a donor forming a Lewis-Acid-donor complex, preferably selected from the group consisting of an aluminium trihalide-donor complex, an alkylaluminium halide-donor complex, an iron trihalide-donor complex, a gallium trihalide-donor complex, a titanium tetrahalide-donor complex, a zinc dihalide-donor complex, a tin dihalide-donor complex, a tin tetrahalide-donor complex, and a boron trihalide-donor complex, said complex comprising, as the donor,
at least one organic compound (II) comprising at least one oxygen or nitrogen atom with at least one lone electron pair, preferably comprising at least one oxygen atom with at least one lone electron pair, very preferably selected from the group consisting of organic compounds with at least one ether function, organic compounds with at least one carboxylic ester function, organic compounds with at least one aldehyde function, organic compounds with at least one keto function, and organic compounds with at least one nitrogen containing heterocyclic ring.

A suitable aluminium trihalide is especially aluminium trifluoride, aluminium trichloride or aluminium tribromide, preferably aluminium trichloride.

A useful alkylaluminium halide is especially a mono(C₁- to C₄-alkyl)aluminium dihalide or a di(C₁- to C₄-alkyl)aluminium monohalide, for example methylaluminium dichloride, ethylaluminium dichloride, iso-butylaluminium dichloride, dimethylaluminium chloride or diethylaluminium chloride, diiso-butylaluminium chloride, preferably ethylaluminium dichloride, iso-butylaluminium dichloride, diethylaluminium chloride or diiso-butylaluminium chloride and very preferably ethylaluminium dichloride and iso-butylaluminium dichloride.

In the context of the present invention C₁ to C₄-alkyl is, for example, methyl, ethyl, propyl, isopropyl, n-butyl, sec-butyl or tert-butyl.

Especially suitable iron trihalides are iron trifluoride, iron trichloride or iron tribromide, preferably iron trichloride.

Especially suitable gallium trihalides are gallium trifluoride, gallium trichloride or gallium tribromide, preferably gallium trichloride.

Especially suitable titanium tetrahalides are titanium tetrafluorides, titanium tetrachlorides or titanium tetrabromides, preferably titanium tetrachlorides.

Especially suitable zinc dihalides are zinc difluorides, zinc dichlorides, or zinc dibromides, preferably zinc dichlorides.

Especially suitable tin dihalides are tin difluorides, tin dichlorides, or tin dibromides, preferably tin dichlorides.

Especially suitable tin tetrahalides are tin tetrafluorides, tin tetrachlorides, or tin tetrabromides, preferably tin tetrachlorides.

Especially suitable boron trihalides are boron trifluoride, boron trichloride, and boron tribromide, preferably boron trifluoride and boron trichloride, and more preferably boron trifluoride.

Among these Lewis Acids, aluminium trihalides, alkylaluminium halides, iron trihalides, titanium tetrahalides, and boron trihalides are preferred.

Very preferred are aluminium trihalides, alkylaluminium halides, iron trihalides, and boron trihalides, particularly preferred are boron trihalides and aluminium trihalides, especially boron trifluorides and aluminium trichloride.

In a preferred embodiment, isobutene or an isobutene-comprising monomer mixture is polymerized in the presence of an alkyl aluminium dichloride-donor complex or an dialkyl aluminium chloride-donor complex effective as a polymerization catalyst, very preferably in the presence of an aluminium trichloride-donor complex or an iron trichloride-donor complex.

In another preferred embodiment, isobutene or an isobutene-comprising monomer mixture is polymerized in the presence of a boron trifluoride-donor complex.

According to the invention the aluminium trihalide-donor complex or alkylaluminium halide-donor complex or the iron trihalide-donor complex or the gallium trihalide-donor complex or the titanium tetrahalide-donor complex or the zinc dihalide-donor complex or the tin dihalide-donor complex or the tin tetrahalide-donor complex or the boron trihalide-donor complex effective as a polymerization catalyst comprises a mixture of the respective metal halide with at least one organic compound (II) as the donor comprising at least one oxygen or nitrogen atom with at least one lone electron pair, preferably comprising at least one oxygen atom with at least one lone electron pair, very preferably selected from the group consisting of organic compounds with at least one ether function, organic compounds with at least one carboxylic ester function, organic compounds with at least one aldehyde function, organic compounds with at least one keto function, and organic compounds with at least one nitrogen containing heterocyclic ring

Compounds (II) comprise at least one oxygen and/or nitrogen atom with at least one lone electron pair, preferably at least one oxygen atom with at least one lone electron pair and very preferably are selected from the group consisting of organic compounds with at least one ether function, organic compounds with at least one carboxylic ester function, organic compounds with at least one aldehyde function, organic compounds with at least one keto function, and organic compounds with at least one nitrogen containing heterocyclic ring.

Solely oxygen containing compounds (II) are preferred over nitrogen-containing compounds (II).

Preferably compound (II) is selected from the group consisting of organic compounds with at least one ether function, organic compounds with at least one carboxylic ester function and organic compounds with at least one keto function, more preferably selected from the group consisting of organic compounds with at least one ether function and organic compounds with at least one carboxylic ester function, very preferably compounds (II) are organic compounds with at least one ether function, and especially organic compounds with exactly one ether function.

Compounds with at least one ether function are also understood to mean acetals and hemiacetals. The ether compound may comprise one or more ether functions, e.g. one, two, three, four or even more ether functions, preferably one or two ether functions and very preferably one ether function.

The mixture of donors may comprise one, two, three, four or even more different compounds (II), preferably compounds with at least one ether function, preferably one or two different compounds and very preferably one compound.

In a preferred embodiment of the present invention, an aluminium trihalide-donor complex or an alkylaluminium halide complex, or an iron trihalide-donor complex, or a gallium trihalide-donor complex or a titanium tetrahalide-donor complex or a zinc dihalide-donor complex or a tin dihalide-donor complex or the tin tetrahalide-donor complex or the boron trihalide-donor complex, very preferably an aluminium trihalide-donor complex or an iron trihalide-donor complex or a boron trihalide-donor complex and especially an aluminium trihalide-donor complex is used, which comprises, as the donor, a mixture of
- at least one dihydrocarbyl ether the general formula R⁸-O-R⁹ in which the variables R⁸ and R⁹ are each independently C₁- to C₂₀-alkyl radicals, preferably C₁- to C₈ alkyl radicals especially C₁- to C₄ alkyl radicals, C₁- to C₂₀-haloalkyl radicals, preferably C₁- to C₈ haloalkyl radicals especially C₁- to C₄ haloalkyl radicals, C₅- to C₈-cycloalkyl radicals, preferably C₅- to Cs-cycloalkyl radicals, C₆- to C₂₀-aryl radicals, especially C₆- to C₁₂ aryl radicals, C₆- to C₂₀-haloaryl radicals, especially C₆- to C₁₂ haloaryl radicals, or C₇- to C₂₀-arylalkyl radicals, especially C₇- to C₁₂-arylalkyl radicals. Preference is given to C₁-to C₄ alkyl radicals, C₁- to C₄ haloalkyl radicals, C₆- to C₁₂ aryl radicals, and C₇- to C₁₂-arylalkyl radicals.

A C₁- to C₈-alkyl radical is a linear or branched alkyl radical having 1 to 8 carbon atoms. Examples thereof are methyl, ethyl, n-propyl, isopropyl, n-butyl, 2-butyl, isobutyl, tert-butyl, pentyl, 1-methylbutyl, 2-methylbutyl, 3-methylbutyl, 2,2-dimethyl-propyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, n-octyl and the constitutional isomers thereof, such as 2-ethylhexyl. Such C₁- to C₈-alkyl radicals may to a small extent also comprise heteroatoms such as oxygen, nitrogen or halogen atoms, for example chlorine, and/or aprotic functional groups, for example carboxyl ester groups, cyano groups or nitro groups.

A C₁- to C₂₀-alkyl radical is a linear or branched alkyl radical having 1 to 20 carbon atoms. Examples thereof are the abovementioned C₁- to C₈-alkyl radicals, and additionally n-nonyl, isononyl, n-decyl, 2-propylheptyl, n-undecyl, n-dodecyl, n-tridecyl, isotridecyl, n-tetradecyl, n-hexadecyl, n-octadecyl and n-eicosyl. Such C₁- to C₂₀-alkyl radicals may to a small extent also comprise heteroatoms such as oxygen, nitrogen or halogen atoms, for example chlorine, and/or aprotic functional groups, for example carboxyl ester groups, cyano groups or nitro groups.

A C₅- to Cs-cycloalkyl radical is a saturated cyclic radical which may comprise alkyl side chains. Examples thereof are cyclopentyl, 2- or 3-methylcyclopentyl, 2,3-, 2,4- or 2,5-dimethylcyclo-pentyl, cyclohexyl, 2-, 3- or 4-methylcyclohexyl, 2,3-, 2,4-, 2,5-, 2,6-, 3,4-, 3,5- or 3,6-dimethyl-cyclohexyl, cycloheptyl, 2-, 3- or 4-methylcycloheptyl, cyclooctyl, 2-, 3-, 4- or 5-methylcyclooctyl. Such C₅- to Cs-cycloalkyl radicals may to a small extent also comprise heteroatoms such as oxygen, nitrogen or halogen atoms, for example chlorine, and/or aprotic functional groups, for example carboxyl ester groups, cyano groups or nitro groups.

A C₆- to C₂₀-aryl radical or a C₆- to C₁₂-aryl radical is preferably optionally substituted phenyl, optionally substituted naphthyl, optionally substituted anthracenyl or optionally substituted phenanthrenyl. Such aryl radicals may be a 1 to 5 aprotic substituents or aprotic functional groups, for example C₁- to C₈-alkyl, C₁- to Ca-haloalkyl such as C₁- to C₈-chloroalkyl or C₁- to C₈-fluoroalkyl, halogens such as chlorine or fluorine, nitro, cyano or phenyl. Examples of such aryl radicals are phenyl, naphthyl, biphenyl, anthracenyl, phenanthrenyl, tolyl, nitrophenyl, chlorophenyl, dichlorophenyl, pentafluorophenyl, pentachlorophenyl, (trifluoromethyl)phenyl, bis(trifluoromethyl)phenyl, (trichloro)methylphenyl and bis(trichloromethyl)phenyl.

A C₇- to C₂₀-arylalkyl radical or a C₇- to C₁₂-arylalkyl radical is preferably optionally substituted C₁- to C₄-alkylphenyl such as benzyl, o-, m- or p-methylbenzyl, 1- or 2-phenyl-ethyl, 1-, 2- or 3-phenylpropyl or 1-, 2-, 3- or 4-phenylbutyl, optionally substituted C₁- to C₄-alkylnaphthyl such as naphthylmethyl, optionally substituted C₁- to C₄-alkylanthracenyl such as anthracenylmethyl, or optionally substituted C₁- to C₄-alkylphenanthrenyl such as phenanthrenylmethyl. Such arylalkyl radicals may bear 1 to 5 aprotic substituents or aprotic functional groups, especially on the aryl moiety, for example C₁- to C₈-alkyl, C₁- to Cs-halo-alkyl such as C₁- to C₈-chloroalkyl or C₁-to C₈-fluoroalkyl, halogen such as chlorine or fluorine, nitro or phenyl.

Haloalkyl and haloaryl mean preferably chloroalkyl or bromoalkyl and chloroaryl or bromo-aryl, very preferably chloroalkyl and chloroaryl. Especially preferred are ω-haioaikyi radicals.

Preferred examples are chloromethyl, 1-chloroeth-1-yl, 2-chloroeth-1-yl, 2-chloroprop-1-yl, 2-chloroprop-2-yl, 3-chloroprop-1-yl, and 4-chlorobut-1-yl.

Preferred examples for chloroaryl are 2-chlorophenyl, 3-chlorophenyl, and 4-chlorophenyl.

The dihydrocarbyl ethers mentioned may be open-chain or cyclic, where the two variables R⁸ and R⁹ in the case of the cyclic ethers may join to form a ring, where such rings may also comprise two or three ether oxygen atoms. Examples of such open-chain and cyclic dihydrocarbyl ethers are dimethyl ether, chloromethyl methyl ether, bis (chloromethyl) ether, diethyl ether, chloromethyl ethyl ether, 2-chloroethyl ethyl ether (CEE), bis (2-chloroethyl) ether (CE), di-n-propyl ether, diisopropyl ether, di-n-butyl ether, di-sec-butyl ether, diisobutyl ether, di-n-pentyl ether, di-n-hexyl ether, di-n-heptyl ether, di-n-octyl ether, di-(2-ethylhexyl) ether, methyl n-butyl ether, methyl sec-butyl ether, methyl isobutyl ether, methyl tert-butyl ether, ethyl n-butyl ether, ethyl sec-butyl ether, ethyl isobutyl ether, ethyl tert-butyl ether, n-propyl-n-butyl ether, n-propyl sec-butyl ether, n-propyl isobutyl ether, n-propyl tert-butyl ether, isopropyl n-butyl ether, isopropyl sec-butyl ether, isopropyl isobutyl ether, isopropyl tert-butyl ether, methyl n-hexyl ether, methyl n-octyl ether, methyl 2-ethylhexyl ether, ethyl n-hexyl ether, ethyl n-octyl ether, ethyl 2-ethylhexyl ether, n-butyl n-octyl ether, n-butyl 2-ethyl-hexyl ether, tetrahydrofuran, tetrahydropyran, 1,2-, 1,3- and 1,4-dioxane, dicyclohexyl ether, diphenyl ether, alkyl aryl ethers, such as anisole and phenetole, ditolyl ether, dixylyl ether and dibenzyl ether.

Furthermore, difunctional ethers such as dialkoxybenzenes, preferably dimethoxybenzenes, very preferably veratrol, and ethylene glycol dialkylethers, preferably ethylene glycol dimethylether and ethylene glycol diethylether, are preferred.

Among the dihydrocarbyl ethers mentioned, diethyl ether, 2-chloroethyl ethyl ether, diisopropyl ether, di-n-butyl ether and diphenyl ether have been found to be particularly advantageous as donors for the aluminium trihalide-donor complexes or the alkylaluminium halide complexes or the iron trihalide-donor complexes or the gallium trihalide-donor complex or the titanium tetrahalide-donor complex or the zinc dihalide-donor complex or the tin dihalide-donor complex or the tin tetrahalide-donor complex or the boron trihalide-donor complex, very preferably the aluminium trihalide-donor complexes or iron trihalide-donor complexes or boron trihalide-donor complex and especially the aluminium trihalide-donor complexes.

In a peferred embodiment dihydrocarbyl ethers with at least one secondary or tertiary dihydrocarbyl group are preferred over dihydrocarbyl groups with primary groups only. Ethers with primary dihydrocarbyl groups are those ethers in which both dihydrocarbyl groups are bound to the ether functional group with a primary carbon atom, whereas ethers with at least one secondary or tertary dihydrocarbyl group are those ethers in which at least one dihydrocarbyl group is bound to the ether functional group with a secondary or tertiary carbon atom.

For the sake of clarity, e.g. diisobutyl ether is deemed to be an ether with primary dihydrocarbyl groups, since the secondary carbon atom of the isobutyl group is not bound to the oxygen of the functional ether group but the hydrocarbyl group is bound via a primary carbon atom.

Preferred examples for ethers with primary dihydrocarbyl groups are diethyl ether, di-n-butyl ether, and di-n-propyl ether.

Preferred examples for ethers with at least one secondary or tertary dihydrocarbyl group are diisopropyl ether, methyl tert-butyl ether, ethyl tert-butyl ether, and anisole.

In addition, particularly advantageous dihydrocarbyl ethers as donors for the aluminium trihalide-donor complexes or the alkylaluminium halide complexes, have been found to be those in which the donor compound has a total carbon number of 3 to 16, preferably of 4 to 16, especially of 4 to 12, in particular of 4 to 8.

In another preferred embodiment halide-substituted ethers are preferred in combination with aluminium halide-donor complex or iron halide-donor complex or boron halide-donor complex.

Organic compounds with at least one carboxylic ester function are preferably hydrocarbyl carboxylates of the general formula R¹⁰-COOR¹¹ in which the variables R¹⁰ and R¹¹ are each independently C₁- to C₂₀-alkyl radicals, especially C₁- to C₈ alkyl radicals, C₅- to Ca-cycloalkyl radicals, C₆- to C₂₀-aryl radicals, especially C₆- to C₁₂ aryl radicals, or C₇- to C₂₀-arylalkyl radicals, especially C₇- to C₁₂-arylalkyl radicals.

Examples of the hydrocarbyl carboxylates mentioned are methyl formate, ethyl formate, n-pro-pyl formate, isopropyl formate, n-butyl formate, sec-butyl formate, isobutyl formate, tert-butyl formate, methyl acetate, ethyl acetate, n-propyl acetate, isopropyl acetate, n-butyl acetate, sec-butyl acetate, isobutyl acetate, tert-butyl acetate, methyl propionate, ethyl propionate, n-propyl propionate, isopropyl propionate, n-butyl propionate, sec-butyl propionate, isobutyl propionate, tert-butyl propionate, methyl butyrate, ethyl butyrate, n-propyl butyrate, isopropyl butyrate, n-bu-tyl butyrate, sec-butyl butyrate, isobutyl butyrate, tert-butyl butyrate, methyl cyclohexanecarboxylate, ethyl cyclohexanecarboxylate, n-propyl cyclohexanecarboxylate, isopropyl cyclohexanecarboxylate, n-butyl cyclohexanecarbo-xylate, sec-butyl cyclohexanecarboxylate, isobutyl cyclohexanecarboxylate, tert-butyl cyclohexanecarboxylate, methyl benzoate, ethyl benzoate, n-pro-pyl benzoate, isopropyl benzoate, n-butyl benzoate, sec-butyl benzoate, isobutyl benzoate, tert-butyl benzoate, methyl phenylacetate, ethyl phenyl acetate, n-propyl phenylacetate, isopropyl phenylacetate, n-butyl phenylacetate, sec-butyl phenyl acetate, isobutyl phenylacetate and tert-butyl phenylacetate. Among the hydrocarbyl carboxylates mentioned, ethyl acetate has been found to be particularly advantageous as a donor for the complexes.

In addition, particularly advantageous hydrocarbyl carboxylates as donors, have been found to be those in which the donor compound has a total carbon number of 3 to 16, preferably of 4 to 16, especially of 4 to 12, in particular of 4 to 8, preference is given in particular to those having a total of 3 to 10 and especially 4 to 6 carbon atoms.

Organic compounds with at least one aldehyde function, preferably exactly one aldehyde function and organic compounds with at least one keto function, preferably exactly one keto function typically have from 1 to 20, preferably from 2 to 10 carbon atoms. Functional groups other than the carbonyl group are preferably absent.

Preferred organic compounds with at least one aldehyde function are those of formula R¹⁰-CHO, in which R¹⁰ has the above-mentioned meaning, very preferably are selected from the group consisting of formaldehyde, acetaldehyde, propionaldehyde, n-butyraldehyde, isobutyraldehyde, and benzaldehyde.

Preferred organic compounds with at least one keto function are those of formula R¹⁰-(C=O)-R¹¹, in which R¹⁰ and R¹¹ have the above-mentioned meaning, very preferably are selected from the group consisting of acetone, methyl ethyl ketone, diethyl ketone, methyl isobutyl ketone, cyclohexanone, acetophenone, and benzophenone. Greatest preference is given to acetone.

Organic compounds with at least one nitrogen containing heterocyclic ring are preferably saturated, partly unsaturated or unsaturated nitrogen-containing five-membered or six-membered heterocyclic rings which comprises one, two or three ring nitrogen atoms and may have one or two further ring heteroatoms from the group of oxygen and sulphur and/or hydrocarbyl radicals, especially C₁- to C₄-alkyl radicals and/or phenyl, and/or functional groups or heteroatoms as substituents, especially fluorine, chlorine, bromine, nitro and/or cyano, for example pyrrolidine, pyrrole, imidazole, 1,2,3- or 1,2,4-triazole, oxazole, thiazole, piperidine, pyrazane, pyrazole, pyridazine, pyrimidine, pyrazine, 1,2,3-, 1,2,4- or 1,2,5-triazine, 1,2,5-oxathiazine, 2H-1,3,5-thiadiazine or morpholine.

However, a very particularly suitable nitrogen-containing basic compound of this kind is pyridine or a derivative of pyridine (especially a mono-, di- or tri-C₁- to C₄-alkyl-substituted pyridine) such as 2-, 3-, or 4-methylpyridine (picolines), 2,3-, 2,4-, 2,5-, 2,6-, 3,4-, 3,5- or 3,6-dimethylpyridine (lutidines), 2,4,6-trimethylpyridine (collidine), 2-, 3,- or 4-tert-butylpyridine, 2-tert-butyl-6-methyl-pyridine, 2,4-, 2,5-, 2,6- or 3,5-di-tert-butylpyridine or else 2-, 3,- or 4-phenylpyridine.

The molar ratio of the donor compounds mentioned to the aluminium trihalide or to the alkylaluminium halide or to the iron trihalide or to the gallium trihalide or to the titanium tetrahalide or to the zinc dihalide or to the tin dihalide or to the tin tetrahalide or to the boron trihalide, in the donor complex generally varies within the range from 0.1:1 to 2.0:1, especially from 0.2:1 to 1.8:1, in particular 0.2:1 to 1.6:1; in most cases it is 0.4:1 to 1.5:1. However, it is also possible to work with a greater excess of the donor compounds, often up to a 10-fold and especially 3-fold molar excess; the excess amount of donor compounds then additionally acts as a solvent or diluent.

The molar ratio of the Lewis Acid mentioned, preferably the boron halide, aluminium trihalide or alkylaluminium halide mentioned to the isobutene monomer used in the case of homopolymerization of isobutene, or to the total amount of the polymerizable monomers used in the case of copolymerization of isobutene, based on each individual functional site of the Lewis Acid mentioned, preferably the boron halide, aluminium trihalide or alkylaluminium halide, is generally from 0.001:1 to 0.2:1, preferably 0.002:1 to 0.1:1, very preferably 0.003:1 to 0.08:1, especially 0.005:1 to 0.05:1, and in particular 0.007:1 to 0.03:1.

Typically, the Lewis Acid-donor complex, preferably the boron trihalide-donor complex, aluminium trihalide-donor complex or the alkylaluminium halide complex, especially the alkyl aluminium dichloride-donor complex or dialkyl aluminium chloride-donor complex, is prepared separately prior to the polymerization from the respective metal halides, preferably the aluminium trihalide or the alkylaluminium halide, especially from anhydrous alkyl aluminium dichloride or an dialkyl aluminium chloride, and the donor compound, and is then - usually dissolved in an inert solvent such as a halogenated hydrocarbon, for example dichlorome-thane, or more preferably in unhalogenated hydrocarbons - added to the polymerization medium. However, in a less preferred embodiment the complex can also be prepared in situ prior to the polymerization.

In case the polymerization catalyst is not fully soluble in the solvent used it may be advantageous to disperse the polymerization catalyst in the inert solvent, e.g. by vigorously stirring the dispersion. The dispersing can be effected in any apparatus suitable for dispersing. Shaking apparatuses such as for example from Skandex may be mentioned by way of example or for example in ultrasonic apparatuses, high pressure homogenizers, 2-, 3-, 4- or 5-roll mills, minimills, Henschel mixers, shaking mills, Ang mills, gear mills, bead mills, wet mills, sand mills, attritors, colloid mills, ultrasonic homogenizers, with Ultra Turrax stirrer and in particular by grinding, for example in 2-, 3-, 4- or 5-roll mills, minimills, shaking mills, Ang mills, gear mills, bead mills, wet mills, sand mills, colloid mills, ball mills, specifically stirred ball mills.

In a preferred embodiment ultrasonication of the Lewis Acid-donor complex, preferably the alumium trihalide-donor complex or the alkylaluminium halide complex, or iron trihalide-donor complexes or boron halide-donor complex in an inert solvent prior to polymerization helps to improve polymerization conversion.

Furthermore, the polymerization is performed with additional use of a mono- or polyfunctional, especially mono-, di- or trifunctional, initiator which is selected from organic hydroxyl compounds, organic halogen compounds and water. It is also possible to use mixtures of the initiators mentioned, for example mixtures of two or more organic hydroxyl compounds, mixtures of two or more organic halogen compounds, mixtures of one or more organic hydroxyl compounds and one or more organic halogen compounds, mixtures of one or more organic hydroxyl compounds and water, or mixtures of one or more organic halogen compounds and water. The initiator may be mono-, di- or polyfunctional, i.e. one, two or more hydroxyl groups or halogen atoms, which start the polymerization reaction, may be present in the initiator molecule. In the case of di- or polyfunctional initiators, telechelic isobutene polymers with two or more, especially two or three, polyisobutene chain ends are typically obtained. Preferred are monofunctional initiators.

Organic hydroxyl compounds which have only one hydroxyl group in the molecule and are suitable as monofunctional initiators include especially alcohols and phenols, in particular those of the general formula R¹²-OH, in which R¹² denotes C₁- to C₂₀-alkyl radicals, especially C₁- to C₈-alkyl radicals, C₅- to Cs-cycloalkyl radicals, C₆- to C₂₀-aryl radicals, especially C₆- to C₁₂-aryl radicals, or C₇- to C₂₀-arylalkyl radicals, especially C₇- to C₁₂-arylalkyl radicals. In addition, the R¹² radicals may also comprise mixtures of the abovementioned structures and/or have other functional groups than those already mentioned, for example a keto function, a nitroxide or a carboxyl group, and/or heterocyclic structural elements.

Typical examples of such organic monohydroxyl compounds are methanol, ethanol, n-propanol, isopropanol, n-butanol, sec-butanol, isobutanol, tert-butanol, n-pentanol, n-hexanol, n-heptanol, n-octanol, 2-ethylhexanol, cyclohexanol, phenol, p-methoxyphenol, o-, m- and p-cresol, benzyl alcohol, p-methoxybenzyl alcohol, 1- and 2-phenylethanol, 1- and 2-(p-methoxyphenyl)ethanol, 1-, 2- and 3-phenyl-1-propanol, 1-, 2- and 3-(p-methoxyphenyl)-1-propanol, 1- and 2-phenyl-2-propanol, 1- and 2-(p-methoxyphenyl)-2-propanol, 1-, 2-, 3- and 4-phenyl-1-butanol, 1-, 2-, 3- and 4-(p-methoxyphenyl)-1-butanol, 1-, 2-, 3- and 4-phe-nyl-2-butanol, 1-, 2-, 3- and 4-(p-me-thoxyphenyl)-2-butanol, 9-methyl-9H-fluoren-9-ol, 1,1-diphenylethanol, 1,1-diphenyl-2-propyn-1-ol, 1,1-diphenylpropanol, 4-(1-hydroxy-1-phenyl-ethyl)benzonitrile, cyclopropyldiphenylmethanol, 1-hydroxy-1,1-diphenylpropan-2-one, benzilic acid, 9-phenyl-9-fluorenol, triphenylmethanol, diphenyl(4-pyridinyl)methanol, alpha,alpha-diphenyl-2-pyridinemethanol, 4-methoxytrityl alcohol (especially polymer-bound as a solid phase), alpha-tert-butyl-4-chloro-4'-methylbenzhydrol, cyclohexyldiphenylmethanol, alpha-(p-tolyl)-benzhydrol, 1,1,2-triphenylethanol, alpha, alpha-diphenyl-2-pyridine-ethanol, alpha,alpha-4-pyridylbenzhydrol N-oxide, 2-fluorotriphenylmethanol, triphenylpro-pargyl alcohol, 4-[(diphenyl)hydroxymethyl]benzonitrile, 1-(2,6-dimethoxyphenyl)-2-methyl-1-phenyl-1-propanol, 1,1,2-triphenylpropan-1-ol and p-anisaldehyde carbinol.

Organic hydroxyl compounds which have two hydroxyl groups in the molecule and are suitable as bifunctional initiators are especially dihydric alcohols or diols having a total carbon number of 2 to 30, especially of 3 to 24, in particular of 4 to 20, and bisphenols having a total carbon number of 6 to 30, especially of 8 to 24, in particular of 10 to 20, for example ethylene glycol, 1,2- and 1,3-propylene glycol, 1,4-butylene glycol, 1,6-hexylene glycol, 1,2-, 1,3- or 1,4-bis(1-hydroxy-1-methylethyl)benzene (o-, m- or p-dicumyl alcohol), bisphenol A, 9,10-di-hydro-9,10-dimethyl-9,10-anthracenediol, 1,1-diphenylbutane-1,4-diol, 2-hydroxytriphenylcarbinol and 9-[2-(hydroxymethyl)-phenyl]-9-fluorenol.

Organic halogen compounds which have one halogen atom in the molecule and are suitable as monofunctional initiators are in particular compounds of the general formula R¹³-Hal in which Hal is a halogen atom selected from fluorine, iodine and especially chlorine and bromine, and R¹³ denotes C₁- to C₂₀-alkyl radicals, especially C₁- to C₈-alkyl radicals, C₅- to Cs-cycloalkyl radicals or C₇- to C₂₀-arylalkyl radicals, especially C₇- to C₁₂-arylalkyl radicals. In addition, the R¹³ radicals may also comprise mixtures of the abovementioned structures and/or have other functional groups than those already mentioned, for example a keto function, a nitroxide or a carboxyl group, and/or heterocyclic structural elements.

Typical examples of such monohalogen compounds are methyl chloride, methyl bromide, ethyl chloride, ethyl bromide, 1-chloropropane, 1-bromopropane, 2-chloropropane, 2-bromopropane, 1-chlorobutane, 1-bromobutane, sec-butyl chloride, sec-butyl bromide, isobutyl chloride, isobutyl bromide, tert-butyl chloride, tert-butyl bromide, 1-chloropentane, 1-bromopentane, 1-chloro-hexane, 1-bromohexane, 1-chloroheptane, 1-bromoheptane, 1-chlorooctane, 1-bromooctane, 1-chloro-2-ethylhexane, 1-bromo-2-ethylhexane, cyclohexyl chloride, cyclohexyl bromide, benzyl chloride, benzyl bromide, 1-phenyl-1-chloroethane, 1-phenyl-1-bromoethane, 1-phenyl-2-chloroethane, 1-phenyl-2-bromoethane, 1-phenyl-1-chloropropane, 1-phenyl-1-bromopropane, 1-phe-nyl-2-chloropropane, 1-phenyl-2-bromopropane, 2-phenyl-2-chloropropane, 2-phenyl-2-bromo-propane, 1-phenyl-3-chloropropane, 1-phenyl-3-bromopropane, 1-phenyl-1-chlorobutane, 1-phenyl-1-bromobutane, 1-phenyl-2-chlorobutane, 1-phenyl-2-bromobutane, 1-phenyl-3-chloro-butane, 1-phenyl-3-bromobutane, 1-phenyl-4-chlorobutane, 1-phenyl-4-bromobutane, 2-phenyl-1-chlorobutane, 2-phenyl-1-bromobutane, 2-phenyl-2-chlorobutane, 2-phenyl-2-bromobutane, 2-phenyl-3-chlorobutane, 2-phenyl-3-bromobutane, 2-phenyl-4-chlorobutane and 2-phenyl-4-bromobutane.

Organic halogen compounds which have two halogen atoms in the molecule and are suitable as difunctional initiators are, for example, 1,3-bis(1-bromo-1-methylethyl)benzene, 1,3-bis(2-chloro-2-propyl)benzene (1,3-dicumyl chloride) and 1,4-bis(2-chloro-2-propyl)benzene (1,4-dicumyl chloride).

The initiator is more preferably selected from organic hydroxyl compounds in which one or more hydroxyl groups are each bonded to an sp³-hybridized carbon atom, organic halogen compounds, in which one or more halogen atoms are each bonded to an sp³-hybridized carbon atom, and water. Among these, preference is given in particular to an initiator selected from organic hydroxyl compounds in which one or more hydroxyl groups are each bonded to an sp³-hybridized carbon atom.

In the case of the organic halogen compounds as initiators, particular preference is further given to those in which the one or more halogen atoms are each bonded to a secondary or especially to a tertiary sp³-hybridized carbon atom.

Preference is given in particular to initiators which may bear, on such an sp³-hydridized carbon atom, in addition to the hydroxyl group, the R¹², R¹³ and R¹⁴ radicals, which are each independently hydrogen, C₁- to C₂₀-alkyl, C₅-to Cs-cycloalkyl, C₆- to C₂₀-aryl, C₇- to C₂₀-alkylaryl or phenyl, where any aromatic ring may also bear one or more, preferably one or two, C₁- to C₄-alkyl, C₁- to C₄-alkoxy, C₁- to C₄-hydroxyalkyl or C₁- to C₄-haloalkyl radicals as substituents, where not more than one of the variables R¹², R¹³ and R¹⁴ is hydrogen and at least one of the variables R¹², R¹³ and R¹⁴ is phenyl which may also bear one or more, preferably one or two, C₁- to C₄-alkyl, C₁-to C₄-alkoxy, C₁- to C₄-hydroxyalkyl or C₁- to C₄-haloalkyl radicals as substituents.

For the present invention, very particular preference is given to initiators selected from water, methanol, ethanol, 1-phenylethanol, 1-(p-methoxyphenyl)ethanol, n-propanol, isopropanol, 2-phenyl-2-propanol (cumene), n-butanol, isobutanol, sec.-butanol, tert-butanol, 1-phenyl-1-chloroethane, 2-phenyl-2-chloropropane (cumyl chloride), tert-butyl chloride and 1,3- or 1,4-bis(1-hydroxy-1-methylethyl)benzene. Among these, preference is given in particular to initiators selected from water, methanol, ethanol, 1-phenylethanol, 1-(p-methoxyphenyl)ethanol, n-pro-panol, isopropanol, 2-phenyl-2-propanol (cumene), n-butanol, isobutanol, sec.-butanol, tert-butanol, 1-phenyl-1-chloroethane and 1,3- or 1,4-bis(1-hydroxy-1-methylethyl)benzene.

The molar ratio of the initiators mentioned to the isobutene monomer used in the case of homopolymerization of isobutene, or to the total amount of the polymerizable monomers used in the case of copolymerization of isobutene, based on each individual functional site of the initiator, is generally from 0.0005:1 to 0.1:1, especially 0.001:1 to 0.075:1, in particular 0.0025:1 to 0.05:1. When water is used as the sole initiator or in combination with organic hydroxyl compounds and/or organic halogen compounds as further initiators, the molar ratio of water to the isobutene monomer used in the case of homopolymerization of isobutene, or to the total amount of the polymerizable monomers used in the case of copolymerization of isobutene, is especially from 0.0001:1 to 0.1:1, in particular 0.0002:1 to 0.05:1, preferably 0.0008:1 to 0.04:1, and very preferably in particular 0.001:1 to 0.03:1.

In a preferred embodiment the amount of initiator in the monomer mixture is not more than 10 wt%, preferably not more than 7.5 wt%, more preferably not more than 5 wt%, even more preferably not more than 3 wt%, and especially not more than 2 wt%.

If water is used as the sole initiator or in combination with organic hydroxyl compounds the amount of initiator in the monomer mixture is not more than 3.2 wt%, preferably not more than 2.5 wt%, more preferably not more than 2 wt%, even more preferably not more than 1.5 wt%, and especially not more than 1 wt%.

A proportion of the initiator molecules added as organic hydroxyl or halogen compounds is incorporated into the polymer chains. The proportion (I_{eff}) of polymer chains which are started by such an incorporated organic initiator molecule may be up to 100%, and is generally 5 to 90%. The remaining polymer chains arise either from water originating from traces of moisture as an initiator molecule, or from chain transfer reactions.

In a further preferred embodiment of the present invention, the polymerization is performed in the presence of 0.01 to 10 mmol, especially of 0.05 to 5.0 mmol, in particular of 0.1 to 1.0 mmol, based in each case on 1 mol of isobutene monomer used in the case of homopolymerization of isobutene, or on 1 mol of the total amount of the polymerizable monomers used in the case of copolymerization of isobutene, of a nitrogen-containing basic compound.

Such a nitrogen-containing basic compound used may be an aliphatic, cycloaliphatic or aromatic amine of the general formula R¹⁴-NR¹⁵R¹⁶, or else ammonia, in which the variables R¹⁴, R¹⁵ and R¹⁶ are each independently hydrogen, C₁- to C₂₀-alkyl radicals, especially C₁- to C₈-alkyl radicals, C₅- to Cs-cycloalkyl radicals, C₆- to C₂₀-aryl radicals, especially C₆- to C₁₂-aryl radicals, or C₇- to C₂₀-arylalkyl radicals, especially C₇- to C₁₂-arylalkyl radicals. When none of these variables is hydrogen, the amine is a tertiary amine. When one of these variables is hydrogen, the amine is a secondary amine. When two of these variables is hydrogen, the amine is a primary amine. When all these variables are hydrogen, the amine is ammonia.

Typical examples of such amines of the general formula R¹⁴-NR¹⁵R¹⁶ are methylamine, ethylamine, n-propylamine, isopropylamine, n-butylamine, tert-butylamine, sec-butylamine, isobutyl-amine, tert-amylamine, n-hexylamine, n-heptylamine, n-octylamine, 2-ethylhexylamine, cyclopentylamine, cyclohexylamine, aniline, dimethylamine, diethylamine, di-n-propylamine, diisopropylamine, di-n-butylamine, di-tert-butylamine, di-sec-butylamine, diisobutylamine, di-tert-amylamine, di-n-hexylamine, di-n-heptylamine, di-n-octylamine, di-(2-ethylhexyl)amine, dicyclopentylamine, dicyclohexylamine, diphenylamine, trimethylamine, triethylamine, tri-n-propyl-amine, tri-isopropylamine, tri-n-butylamine, tri-tert-butylamine, tri-sec-butylamine, tri-isobutyl-amine, tri-tert-amylamine, tri-n-hexylamine, tri-n-heptylamine, tri-n-octylamine, tri-(2-ethylhexyl)-amine, tricyclopentylamine, tricyclohexylamine, triphenylamine, dimethylethylamine, methyl-n-butylamine, N-methyl-N-phenylamine, N,N-dimethyl-N-phenylamine, N-methyl-N,N-diphenylamine or N-methyl-N-ethyl-N-n-butylamine.

In addition, such a nitrogen-containing basic compound used may also be a compound having a plurality of, especially having two or three, nitrogen atoms and having 2 to 20 carbon atoms, where these nitrogens may each independently bear hydrogen atoms or aliphatic, cycloaliphatic or aromatic substituents. Examples of such polyamines are 1,2-ethylenediamine, 1,3-propylene-diamine, 1,4-butylenediamine, diethylenetriamine, N-methyl-1,2-ethylenediamine, N,N-dimethyl-1,2-ethylenediamine, N,N'-dimethyl-1,2-ethylenediamine or N,N-dimethyl-1,3-propylenediamine.

However, a suitable nitrogen-containing basic compound of this kind is especially a saturated, partly unsaturated or unsaturated nitrogen-containing five-membered or six-membered heterocyclic ring which comprises one, two or three ring nitrogen atoms and may have one or two further ring heteroatoms from the group of oxygen and sulphur and/or hydrocarbyl radicals, especially C₁- to C₄-alkyl radicals and/or phenyl, and/or functional groups or heteroatoms as substituents, especially fluorine, chlorine, bromine, nitro and/or cyano, for example pyrrolidine, pyrrole, imidazole, 1,2,3- or 1,2,4-triazole, oxazole, thiazole, piperidine, pyrazane, pyrazole, pyridazine, pyrimidine, pyrazine, 1,2,3-, 1,2,4- or 1,2,5-triazine, 1,2,5-oxathiazine, 2H-1,3,5-thiadiazine or morpholine.

However, a very particularly suitable nitrogen-containing basic compound of this kind is pyridine or a derivative of pyridine (especially a mono-, di- or tri-C₁- to C₄-alkyl-substituted pyridine) such as 2-, 3-, or 4-methylpyridine (picolines), 2,3-, 2,4-, 2,5-, 2,6-, 3,4-, 3,5- or 3,6-dimethylpyridine (lutidines), 2,4,6-trimethylpyridine (collidine), 2-, 3,- or 4-tert-butylpyridine, 2-tert-butyl-6-methyl-pyridine, 2,4-, 2,5-, 2,6- or 3,5-di-tert-butylpyridine or else 2-, 3,- or 4-phenylpyridine.

It is possible to use a single nitrogen-containing basic compound or mixtures of such nitrogen-containing basic compounds.

The polymerization of the isobutene or of the isobutenic starting material generally proceeds spontaneously when the Lewis Acid, preferably the Lewis Acid-donor complex, more preferably the boron trihalide-donor complex, aluminium trihalide-donor complex or the alkylaluminium halide complex, especially the alkyl aluminium dichloride-donor complex or dialkyl aluminium chloride-donor complex, is contacted with the isobutene or the isobutenic monomer mixture at the desired reaction temperature. The procedure here may be to initially charge the monomers, optionally in the diluent, to bring it to reaction temperature and then to add the Lewis Acid-donor complex, preferably the boron trihalide-donor complex, aluminium trihalide-donor complex or the alkylaluminium halide complex, especially the alkyl aluminium dichloride-donor complex or dialkyl aluminium chloride-donor complex. The procedure may also be to initially charge the Lewis Acid-donor complex, preferably the boron trihalide-donor complex, aluminium trihalide-donor complex or the alkylaluminium halide complex, especially the alkyl aluminium dichloride-donor complex or dialkyl aluminium chloride-donor complex, optionally in the diluent, and then to add the monomers. In that case, the start of polymerization is considered to be that time at which all reactants are present in the reaction vessel.

To prepare isobutene copolymers, the procedure may be to initially charge the monomers, optionally in the diluent, and then to add the Lewis Acid-donor complex, preferably the boron trihalide-donor complex, aluminium trihalide-donor complex or the alkylaluminium halide complex, especially the alkyl aluminium dichloride-donor complex or dialkyl aluminium chloride-donor complex. The reaction temperature can be established before or after the addition of the Lewis Acid-donor complex, preferably boron trihalide-donor complex, the aluminium trihalide-donor complex or the alkylaluminium halide complex, especially of the alkyl aluminium dichloride-donor complex or dialkyl aluminium chloride-donor complex. The procedure may also be first to initially charge only one of the monomers, optionally in the diluent, then to add the Lewis Acid-donor complex, preferably the boron trihalide-donor complex, aluminium trihalide-donor complex or the alkylaluminium halide complex, especially the alkyl aluminium dichloride-donor complex or dialkyl aluminium chloride-donor complex, and to add the further monomer(s) only after a certain time, for example when at least 60%, at least 80% or at least 90% of the monomer has been converted. Alternatively, the Lewis Acid-donor complex, preferably the boron trihalide-donor complex, aluminium trihalide-donor complex or the alkylaluminium halide complex, especially the alkyl aluminium dichloride-donor complex or dialkyl aluminium chloride-donor complex, can be initially charged, optionally in the diluent, then the monomers can be added simultaneously or successively, and then the desired reaction temperature can be established. In that case, the start of polymerization is considered to be that time at which the Lewis Acid-donor complex, preferably the boron trihalide-donor complex, aluminium trihalide-donor complex or the alkylaluminium halide complex, especially the alkyl aluminium dichloride-donor complex or dialkyl aluminium chloride-donor complex, and at least one of the monomers are present in the reaction vessel.

In addition to the batchwise procedure described here, the polymerization in the process according to the invention can also be configured as a continuous process. In this case, the feedstocks, i.e. the monomer(s) to be polymerized, optionally the diluent and optionally the Lewis Acid-donor complex, preferably the boron trihalide-donor complex, aluminium trihalide-donor complex or the alkylaluminium halide complex, especially the alkyl aluminium dichloride-donor complex or dialkyl aluminium chloride-donor complex, are supplied continuously to the polymerization reaction, and reaction product is withdrawn continuously, such that more or less steady-state polymerization conditions are established in the reactor. The monomer(s) to be polymerized can be supplied as such, diluted with a diluent or solvent, or as a monomer-containing hydrocarbon stream.

The Lewis Acid-donor complex, preferably the boron trihalide-donor complex, aluminium trihalide-donor complex effective as a polymerization catalyst or the alkylaluminium halide complex, especially alkyl aluminium dichloride-donor complex or dialkyl aluminium chloride-donor complex, is generally present in dissolved, dispersed or suspended form in the polymerization medium. Supporting of the Lewis Acid-donor complex, preferably of the boron trihalide-donor complex, aluminium trihalide-donor complex or of the alkylaluminium halide complex, especially of alkyl aluminium dichloride-donor complex or dialkyl aluminium chloride-donor complex, on customary support materials is also possible. Suitable reactor types for the polymerization process of the present invention are typically stirred tank reactors, loop reactors and tubular reactors, but also fluidized bed reactors, stirred tank reactors with or without solvent, fluid bed reactors, continuous fixed bed reactors and batchwise fixed bed reactors (batchwise mode).

In principle, all types of discontinuous or continuously operated reactors suitable for such liquid-phase polymerizations can be used as polymerization reactors for the process according to the invention, such as stirred tanks, stirred tank cascades, tube reactors or loop reactors. If polymerization is carried out according to the method according to the invention at or above the boiling temperature of any inert diluent or monomer to be polymerized, it is preferably carried out in pressure vessels, for example in autoclaves or in pressure reactors.

Furthermore, it is important that polymerizable compounds are moved in containers, for example by stirring, natural circulation or pumping around (forced circulation).

In the process according to the invention, the boron trihalide-donor complex or aluminium trihalide-donor complex effective as a polymerization catalyst or the alkylaluminium halide complex, or the iron trihalide-donor complex, or the gallium trihalide-donor complex, or the titanium tetrahalide-donor complex, or the zinc dihalide-donor complex, or the tin dihalide-donor complex, or the tin tetrahalide-donor complex or the boron trihalide-donor complex, especially the alkyl aluminium dichloride-donor complex or dialkyl aluminium chloride-donor complex, is generally used in such an amount that the molar ratio of the metal in the Lewis Acid donor complex, preferably the aluminium in the aluminium trihalide-donor complex or alkylaluminium halide complex, especially in the alkyl aluminium dichloride-donor complex or dialkyl aluminium chloride-donor complex, to isobutene in the case of homopolymerization of isobutene, or to the total amount of the polymerizable monomers used in the case of copolymerization of isobutene, is in the range from 1:5 to 1:5000, preferably from 1:10 to 1:5000, especially 1:15 to 1:1000, in particular 1:20 to 1:250.

In a preferred embodiment of the present invention the reaction conditions are chosen that the conversion of the monomers is at least 80%, preferably at least 85%, and more preferably at least 90%.

According to the invention the polymerization is carried out as a polymerization in bulk or in solution.

The polymerization in the process according to the invention is preferably performed in the presence of an inert diluent. The inert diluent used should be suitable for reducing the increase in the viscosity of the reaction solution which generally occurs during the polymerization reaction to such an extent that the removal of the heat of reaction which evolves can be ensured. Suitable diluents are those solvents or solvent mixtures which are inert toward the reagents used. Suitable diluents are, for example, aliphatic hydrocarbons such as n-butane, n-pentane, n-hexane, n-heptane, n-octane and isooctane, cycloaliphatic hydrocarbons such as cyclopentane and cyclohexane, aromatic hydrocarbons such as benzene, toluene and the xylenes, and halogenated hydrocarbons, especially halogenated aliphatic hydrocarbons, such as methyl chloride, dichloromethane and trichloromethane (chloroform), 1,1-dichloroethane, 1,2-dichloroethane, trichloroethane and 1-chlorobutane, and also halogenated aromatic hydrocarbons and alkylaromatics halogenated in the alkyl side chains, such as chlorobenzene, monofluoromethylbenzene, difluoromethylbenzene and trifluoromethylbenzene, and mixtures of the aforementioned diluents. The diluents used, or the constituents used in the solvent mixtures mentioned, are also the inert components of isobutenic C4 hydrocarbon streams. A non-halogenated solvent is preferred over the list of halogenated solvents.

The inventive polymerization may be performed in a halogenated hydrocarbon, especially in a halogenated aliphatic hydrocarbon, or in a mixture of halogenated hydrocarbons, especially of halogenated aliphatic hydrocarbons, or in a mixture of at least one halogenated hydrocarbon, especially a halogenated aliphatic hydrocarbon, and at least one aliphatic, cycloaliphatic or aromatic hydrocarbon as an inert diluent, for example a mixture of dichloromethane and n-hexane, typically in a volume ratio of 10:90 to 90:10, especially of 50:50 to 85:15. Prior to use, the diluents are preferably freed of impurities such as water, carboxylic acids or mineral acids, for example by adsorption on solid adsorbents such as activated carbon, molecular sieves or ion exchangers.

In a preferred embodiment, the inventive polymerization is performed in halogen-free aliphatic or especially halogen-free aromatic hydrocarbons, especially toluene. For this embodiment, water in combination with the organic hydroxyl compounds mentioned and/or the organic halogen compounds mentioned, or especially as the sole initiator, have been found to be particularly advantageous.

In another preferred embodiment, the inventive polymerization is performed in halogen-free aliphatic or cycloaliphatic, preferably aliphatic hydrocarbons, especially hexane, pentane, heptane, cyclohexane, cyclopentane, and mixtures comprising them.

The polymerization in the process according to the invention is preferably performed under substantially aprotic and especially under substantially anhydrous reaction conditions. Substantially aprotic and substantially anhydrous reaction conditions are understood to mean that, respectively, the content of protic impurities and the water content in the reaction mixture are less than 50 ppm and especially less than 5 ppm. In general, the feedstocks will therefore be dried before use by physical and/or chemical measures. More particularly, it has been found to be useful to admix the aliphatic or cycloaliphatic hydrocarbons used as solvents, after customary prepurification and predrying with an organometallic compound, for example an organolithium, organomagnesium or organoaluminium compound, in an amount which is sufficient to substantially remove the water traces from the solvent. The solvent thus treated is then preferably condensed directly into the reaction vessel. It is also possible to proceed in a similar manner with the monomers to be polymerized, especially with isobutene or with the isobutenic mixtures. Drying with other customary desiccants such as molecular sieves or predried oxides such as aluminium oxide, silicon dioxide, calcium oxide or barium oxide is also suitable. The halogenated solvents for which drying with metals such as sodium or potassium or with metal alkyls is not an option are freed of water or water traces with desiccants suitable for that purpose, for example with calcium chloride, phosphorus pentoxide or molecular sieves. It is also possible in an analogous manner to dry those feedstocks for which treatment with metal alkyls is likewise not an option, for example vinylaromatic compounds. Even if some or all of the initiator used is water, residual moisture should preferably be very substantially or completely removed from solvents and monomers by drying prior to reaction, in order to be able to use the water initiator in a controlled, specified amount, as a result of which greater process control and reproducibility of the results are obtained.

To stop the reaction, the reaction mixture is preferably deactivated, for example by adding a protic compound, especially by adding water, alcohols such as methanol, ethanol, n-propanol and isopropanol or mixtures thereof with water, or by adding an aqueous base, for example an aqueous solution of an alkali metal or alkaline earth metal hydroxide such as sodium hydroxide, potassium hydroxide, magnesium hydroxide or calcium hydroxide, an alkali metal or alkaline earth metal carbonate such as sodium, potassium, magnesium or calcium carbonate, or an alkali metal or alkaline earth metal hydrogencarbonate such as sodium, potassium, magnesium or calcium hydrogencarbonate.

The process according to the invention serves to prepare high-reactivity isobutene homo- or copolymers with a content of terminal vinylidene double bonds (α-double bonds) and other reactive double bonds, such as β-double bonds per polyisobutene chain end of at least 60, preferably at least 70 and very preferably at least 75 mol%. More particularly, it also serves to prepare high-reactivity isobutene copolymers which are formed from isobutene and at least one vinylaromatic monomer, especially styrene, and have a content of terminal vinylidene double bonds (α-double bonds) and other reactive double bonds, such as β-double bonds per polyisobutene chain end of at least 60, preferably at least 70 mol%, preferably of at least 75 mol%. To prepare such copolymers of isobutene and at least one vinylaromatic monomer, especially styrene, isobutene or an isobutenic hydrocarbon cut is copolymerized with the at least one vinylaromatic monomer in a weight ratio of isobutene to vinylaromatic of 5:95 to 95:5, especially of 30:70 to 70:30.

The high-reactivity isobutene homo- or copolymers prepared by the process according to the invention and specifically the isobutene homopolymers preferably have a polydispersity (PDI = M_{w}/Mₙ) of 1.05 to less than 3.5, preferably of 1.05 to less than 3.0, preferably of 1.05 to less than 2.5, preferably of 1.05 to 2.3, more preferably of 1.05 to 2.0 and especially of 1.1 to 1.85. Typical PDI values in the case of an optimal process regime are 1.2 to 1.7.

The method according to the invention is operated at different reaction temperatures depending on the desired molar weight of the polyisobutene:
In a preferred embodiment of the present invention, when a polyisobutene with a number average molecular weight Mn of 350 to 10000, preferably 500 to 5000 is targeted, the reaction temperature in the reaction mixture is preferably from 5 to 25 °C.

In particular, this homo- or copolymer is a highly reactive polyisobutene with a content of terminal vinylidene groups of at least 70 mol%, preferably at least 80 mol%, preferably at least 90 mol%.

In an alternative preferred embodiment of the present invention, when a number average molecular weight Mn of 10000 to 100000 is targeted, the reaction temperature in the reaction mixture is preferably from 10 to 30 °C.

In an alternative preferred embodiment of the present invention, when a number average molecular weight Mn of at least 100000 to 1000000 is sought, the reaction temperature in the reaction mixture is preferably from 50 to 90 °C.

As a rule, the average residence time in the reaction system without mixing, e.g. by circulation, stirring or backmixing, should be less than 2 hours, preferably less than 90 minutes and preferably less than 60 minutes.

Polymerization is usually carried out at a pressure of 700 mbar to 20 bar, especially at a pressure of 1 bar to 10 bar, especially at a pressure of 1.2 bar to 7 bar. Overpressure is usually advantageous with the C4 hydrocarbon mixture used and with some inert diluents that may be used.

Another object of the present invention is the polyisobutene obtainable preferably obtained by the process according to the present invention.

Highly reactive polyisobutene of low or medium molecular weight may preferably be used as starting material for subsequent chemical reactions, e.g. subsequent hydroformylation, thermal ene-reaction with maleic anhydride, or *Friedel-Crafts* alkylation of aromatic compounds.

Medium and high molecular weight polyisobutene not exhibiting a high reactivity is less susceptible to weathering and more stable against oxidation or thermal degradation. Therefore, compositions comprising such polyisobutenes are especially useful in sealants, adhesives, coatings or roofings.

In an embodiment of the present invention, the process comprises the steps of:
a) feeding into a reaction vessel a stream comprising linear and/or branched C₅-C₈ primary alcohol which has at least one hydrogen atom attached to the carbon atom adjacent to the one bearing the alcohol group,
b) providing a catalyst comprising at least one catalytically active metal selected from platinum (Pt), palladium (Pd), nickel (Ni), Chromium (Cr), Molybdenum (Mo), Ruthenium (Ru), or combinations thereof, and
c) subjecting the primary alcohol to a de-hydroformulation in the presence of the catalyst of step b) yielding olefin having one carbon atom less than the starting alcohol,
d) optionally purifying the reaction product from step c) yielding an olefin-containing product stream, and
e) polymerising the olefin obtained in step c) or d) (if applicable) optionally together with olefin from other sources in the presence of at least one Lewis Acid and in the presence of at least one initiator,
wherein the linear and/or branched C₅-C₈ primary alcohol comprises 3-Methyl 1-butanol and the olefin comprises isobutene, and
wherein linear or branched C₅-C₈ alcohol is converted to at least one lower hydrocarbon having C₄-C₇ carbon atoms at a yield of at least about 80 wt%. of the maximum theoretical molar yield.

In an embodiment of the present invention, the process comprises the steps of:
a) feeding into a reaction vessel a stream comprising linear and/or branched C₅-C₈ primary alcohol which has at least one hydrogen atom attached to the carbon atom adjacent to the one bearing the alcohol group,
b) providing a catalyst comprising at least one catalytically active metal selected from platinum (Pt), palladium (Pd), nickel (Ni), Chromium (Cr), Molybdenum (Mo), Ruthenium (Ru), or combinations thereof, further comprising at least one promoter selected from Tin (Sn), Silver (Ag), Iridium (Ir), Rhenium (Re), or combinations thereof, wherein the at least one promoter is present in an amount in the range from 0.005 wt% to 5.0 wt%, with respect to total weight of the catalyst, and
c) subjecting the primary alcohol to a de-hydroformulation in the presence of the catalyst of step b) yielding an olefin having one carbon atom less than the starting alcohol, wherein step c) is conducted at a partial pressure in the range of 0.02 to 4.0 bar, and at a temperature in the range of 200 °C to 450 °C, preferably 250 °C to 450 °C, more preferably 300 to 450 °C, even more preferably 300 °C to 400 °C, especially 300 °C to 350 °C,
d) optionally purifying the reaction product from step c) yielding an olefin-containing product stream, and
e) polymerising the olefin obtained in step c) or d) (if applicable) optionally together with olefin from other sources in the presence of at least one Lewis Acid and in the presence of at least one initiator,
wherein the linear and/or branched C₅-C₈ primary alcohol comprises 3-Methyl 1-butanol and the olefin comprises isobutene, and
wherein linear or branched C₅-C₈ alcohol which is isoamyl alcohol is converted to at least one lower hydrocarbon having C₄-C₇ carbon atom which is isobutylene, at a yield of at least about 80 wt.% of the maximum theoretical molar yield.

Advantages of the present invention
1. The process according to the invention enables the preparation of olefins with high yield and high selectivity under mild conditions, both of temperature and pressure, while requiring only moderate to low amounts of catalyst.
2. The process can be conducted with no or low amounts of organic solvent, thus avoiding or minimizing environmentally problematic waste stream.
3. A further advantage of the process of the invention is that the desired olefin is obtained in a high concentration in the reaction mixture, thus minimizing down-stream isolation steps.
4. The process of the present invention enables the formation of olefins in comparable yield and purity irrespective of the source of the primary alcohol used as the starting material. (Pure primary alcohol or alcohol isolated from fusel oil). Thus, the impurities of the starting material do not hamper the yield and purity of the final product (olefin in this case).
5. The process according to the present invention uses readily available reagents and thereby reduces the overall cost of the process, thereby making it industrially viable process which can be easily scaled up.
6. The process according to the present invention, uses 3-methyl-1-butanol from fusel oil as the starting material and using the de-hydroformulation to isobutene. Although the individual steps are known reaction, they have not been described for this particular substrate, and they have not been combined as a means of obtaining olefin (isobutene) with a pMC> 90% and thus can be considered to be inventive.
7. The olefin (isobutene) that is obtained would also have a pMC value greater than 90 which can be deduced from the fact that the input stream comprising linear or branched C₅-C₈ primary alcohol has a pMC value greater than 90 (which is obtained in natural sources -namely the fusel oil).
8. The olefin (isobutene) that is obtained can be used in the preparation of polyisobutene homo- or copolymers either as a predominatly or preferably pure monomer from renewable sources or together with isobutene from fossil sources.

### Embodiments

In the following, there is provided a list of embodiments to further illustrate the present disclosure without intending to limit the disclosure to the specific embodiments listed below.
1. A process for preparing a lower olefin with 4 to 7 carbon atoms, comprising the steps of:
   a) feeding into a reaction vessel a stream comprising linear and/or branched C₅-C₈ primary alcohol which has at least one hydrogen atom attached to the carbon atom adjacent to the one bearing the alcohol group,
   b) providing a catalyst comprising at least one catalytically active metal selected from platinum (Pt), palladium (Pd), nickel (Ni), Chromium (Cr), Molybdenum (Mo), Ruthenium (Ru), or combinations thereof, and
   c) subjecting the primary alcohol to a de-hydroformulation in the presence of the catalyst of step b) yielding an olefin having one carbon atom less than the starting alcohol.
2. The process according to embodiment 1, wherein the stream comprising linear and/or branched C₅- C₈ primary alcohol which has at least one hydrogen atom attached to the carbon atom adjacent to the one bearing the alcohol group has a pMC at least greater than 90, preferably pMC values of at least greater than 95, preferably pMC values of at least greater than 98, more preferably pMC values of at least greater than 99, more preferably pMC values of at least about 100, inclusive of all values and subranges there- between, when measured by a method as described in the ASTM norm D6866.
3. The process according to embodiment 2, wherein the stream comprising linear or branched C₅-C₈ primary alcohol which has at least one hydrogen atom attached to the carbon atom adjacent to the one bearing the alcohol group is obtained from natural sources.
4. The process according to embodiment 2, wherein the stream comprising linear or branched C₅-C₈ primary alcohol which has at least one hydrogen atom attached to the carbon atom adjacent to the one bearing the alcohol group is obtained from fermentation process.
5. The process according to embodiment 1, wherein the stream comprising linear and/or branched C₅-C₈ primary alcohol has at least one hydrogen atom attached to the carbon atom adjacent to the one bearing the alcohol group that is the alpha carbon atom.
6. The process according to embodiment 5, wherein the stream comprising the linear and/or branched C₅-C₈ primary alcohol has at least one hydrogen atom attached to the carbon atom adjacent to the one bearing the alcohol group that is the alpha carbon atom and the beta carbon atom (i.e the carbon atom attached to the first carbon atom adjacent to the one bearing the alcohol group) also has at least one hydrogen atom.
7. The process according to any of the embodiments 1 to 6, wherein the linear or branched C₅-C₈ primary alcohol which has at least one hydrogen atom attached to the carbon atom adjacent to the one bearing the alcohol group is selected from 2-methyl-1-butanol (active amyl alcohol), 3-methylbutan-1-ol (isoamyl alcohol, (isopentanol), n-pentanol, n-hexanol, 2-methylpentanol, n-heptanol, n-octanol, 2-ethylhexanol.
8. The process according to any of the embodiments 1 to 7, wherein the linear or branched C₅-C₈ primary alcohol which has at least one hydrogen atom attached to the carbon atom adjacent to the one bearing the alcohol group is 3-methylbutan-1-ol (isoamyl alcohol, isopentanol).
9. The process according to any of the embodiments 1 to 8, wherein the stream comprises linear or branched C₅-C₈ primary alcohol which has at least one hydrogen atom attached to the carbon atom adjacent to the one bearing the alcohol group at a concentration in the range of 60wt%-99wt %, preferably in the range of 60wt%-95wt %, preferably in the range of 60wt%-90wt %, preferably in the range of 60wt%-85wt %, preferably in the range of 60wt%-80wt %, more preferably in the range of 65wt%-80wt %, even more preferably in the range of 70wt%-80wt %.
10. The process according to embodiment 1, wherein in step (b), a catalyst is provided comprising at least one catalytically active metal selected from platinum (Pt), palladium (Pd), nickel (Ni), Chromium (Cr), Molybdenum (Mo), Ruthenium (Ru), or combinations thereof.
11. The process according to embodiment 10, wherein the catalyst in step b) further comprises at least one promoter selected from Tin (Sn), Silver (Ag), Iridium (Ir), Rhenium (Re), or combinations thereof, wherein the at least one promoter is present in an amount in the range from 0.005 wt% to 5.0 wt%, with respect to total weight of the catalyst.
12. The process according to embodiment 11, wherein in the catalyst in step b) the at least one catalytically active metal is be supported on at least one carrier on which the catalytically active metal is supported, wherein the carrier is selected from a non-acidic material selected from calcium oxide, titanium oxide, beryllia, magnesia, thoria, zirconia, alumina, silicon carbide, quartz or combinations thereof.
13. The process according to any of the previous embodiments, wherein step c) is conducted at a partial pressure in the range of 0.02 to 4.0 bar, and at a temperature in the range of 200 °C to 450 °C, preferably 250 °C to 450 °C, more preferably 300 to 450 °C, even more preferably 300 °C to 400 °C, especially 300 °C to 350 °C.
14. The process according to embodiment 13, wherein the feed stream in step (c) is fed at a gas hourly space velocity (GHSV) in the range from 100 h⁻¹ to 15000 h⁻¹.
15. The process according to embodiment 13 or 14, wherein in step c) the amount of the at least one primary alcohol in the feed stream is in the range from 2.0 wt% to 100.0 wt% based on the total weight of the feed stream.
16. The process according to any one of the embodiments 13 to 15, wherein the feed stream further comprises at least one inert gas selected from N₂, CO₂, CH₄ or Ar.
17. The process according to any one of the prvious embodiments, wherein the lower hydrocarbon having C₄-C₇ carbon atoms is selected from is 1-butene, isobutylene, 1-pentene, isopentene, 1- hexene, 2-hexene, 3- hexene, 4-methyl-1-pentene.
18. The process according to any one of the embodiment 1 to 16, wherein the lower hydrocarbon having C₄-C₇ carbon atoms is isobutylene.
19. The process according to any one of the previous embodiments, wherein linear or branched C₅-C₈ primary alcohol which has at least one hydrogen atom attached to the carbon atom adjacent to the one bearing the alcohol group is converted to at least one lower hydrocarbon having C₄-C₇carbon atoms at a yield of at least about 80 wt%. of the maximum theoretical molar yield,

### Examples

The presently claimed invention is illustrated in detail by non-restrictive working examples which follow. More particularly, the test methods specified hereinafter are part of the general disclosure of the application and are not restricted to the specific working examples.

### MATERIALS AND METHOD

Butanol pure (boiling point 117.7 °C).
Isoamyl alcohol pure (boiling point 131 °C).
Fusel alcohol (70% isoamyl alcohol + others).
Density of Pt/ZrO₂ catalyst = 1.18 g/cm³.

The reactions were carried out using a highly flexible reactor (HiFlexT: R0027) equipped with 16 individual reactors, each with a heater capable of achieving temperatures up to 750°C (850 °C), at a pressure of up to 100 bar. Each reactor had capability of holding a catalyst up to 2.0 ml.

The powdered catalysts were sieved (or screened) prior to the catalytic testing experiments. For the catalytic testing experiments a split fraction in the range of 100 to 350 µm was used.

Examples were conducted at three different reactor filling levels (or filling volumes), which were 0.25, 0.5 and 1.0 ml. The details are listed in the respective Tables. This means that the amount of catalyst which was used for an individual experiment was in the range of 0.295 to 1.18 g in case of Pt-metal on zirconium oxide (which has a density of 1.18 g/cm³). To keep the catalyst beds on a constant level, the catalyst material were diluted with inert particles in such way that all reactors had a constant filling volume.

The components of product feed/stream were detected and quantified using a Gas Chromatography system. The Gas Chromatography system was calibrated using GC standard/s for prenol, prenal, and isoprenal. The Gas Chromatography system was calibrated for products such as: CO, H₂, C₁-C₆ alkanes and alkenes, isoamyl alcohol, 3- methyl butanal, isobutene, isoprenol, and isoprene.

The following examples are included to further illustrate the invention.

A series of catalysts were used for the consecutive dehydrogenation / retro-dehydroformylation reaction in a single fixed bed reactor. The catalysts used for these reactions contained 0.005 wt% to 10.0 wt% of catalytically active metal (based on total weight of the catalyst). Optionally, the catalyst further contained sufficient amount of a promoter in an amount from 0.005 wt% to 5.0 wt%, with respect to total weight of the catalyst. The catalysts were charged to a conventional vapor phase reactor having temperature control, inlet/s for introduction of feed including inert/carrier/hydrogen gas and outlets for withdrawal of products. The reactions were carried out by varying temperatures from 200 to 450 °C, and weight ratios of alcohol in the feed from 2.0 wt% to 88.0 wt% (based on total weight of the feed). The yield of the olefin for each catalyst was determined at different conversion levels.

The data is summarized in the Figures and showed that the catalyst containing a 1.0 wt% Pt / 0.5 wt% Sn / ZrO₂ was unique.

### n-Butanol to propene:

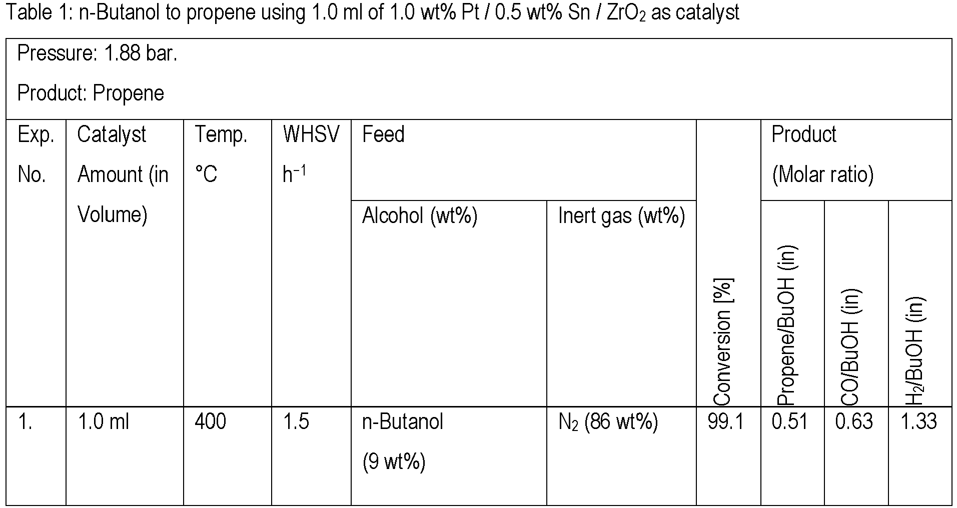

From Table 1, it is evident that by using 1.0 ml of 1.0 wt% Pt / 0.5 wt% Sn / ZrO₂ as catalyst up to 100% conversion of butanol was achieved.

Thus, a carbon selectivity of 61.0 % for propene and 35.0 % for CO was achieved (theoretical up to 75% and 25% respectively).

### Isoamyl alcohol to isobutene:

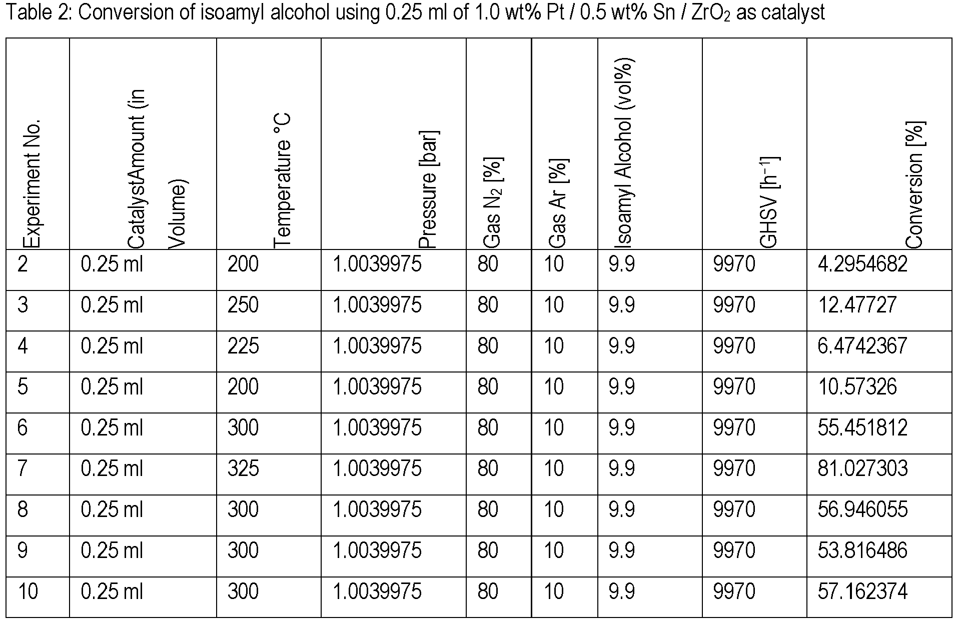

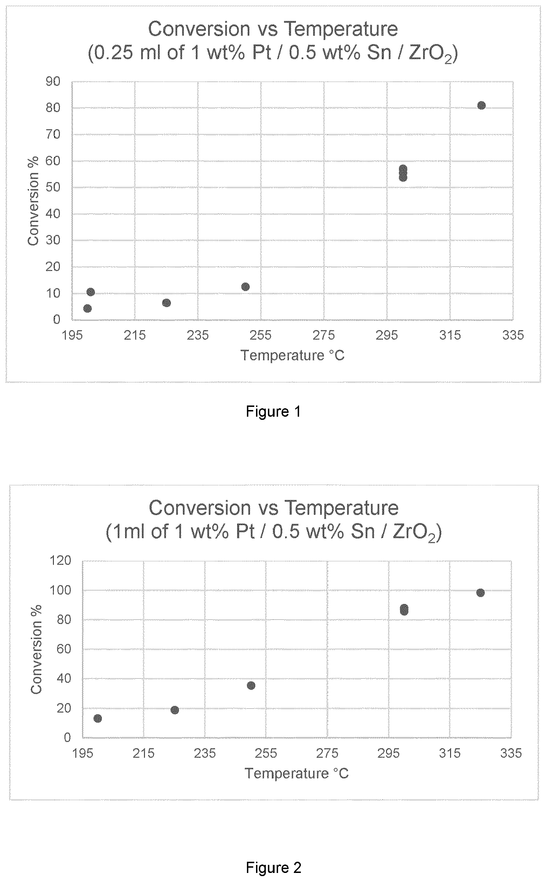

From Table 2, it is evident that on passing a gas feed of 9.9 vol% isoamyl alcohol at a GHSV of 9970 h⁻¹ over 0.25 ml of 1.0 wt% Pt / 0.5 wt% Sn / ZrO₂ catalyst, 4.0% to 81.0% conversion of isoamyl alcohol was achieved at a pressure of 1.0 bar, and at a temperature in the range of 200 to 325 °C. It is also evident that highest conversion (81.0%) was achieved at a temperature of 325 °C. The results of Table 2
are illustrated graphically in Figure 1.

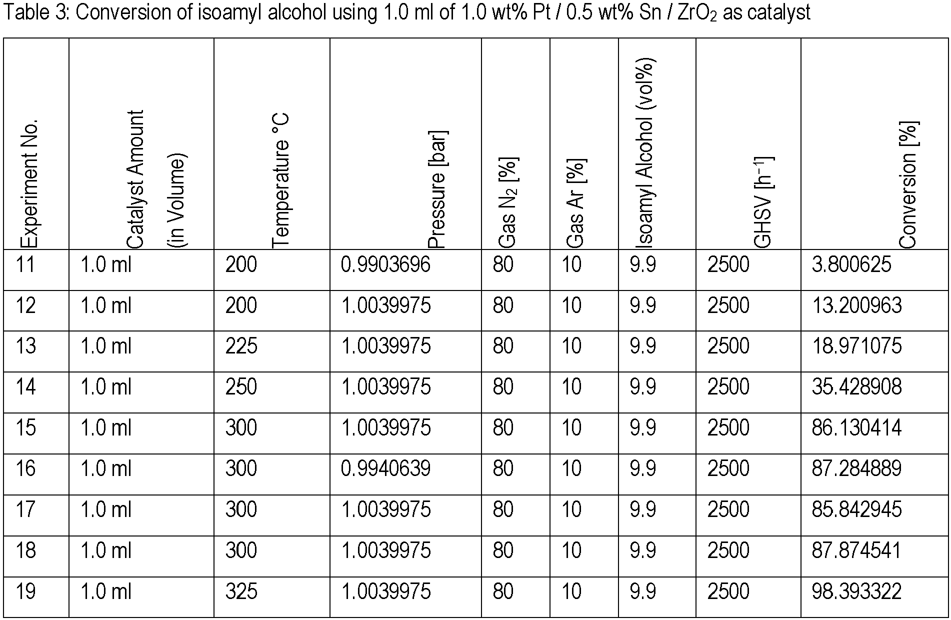

From Table 3, it is evident that on passing a gas feed of 9.9 vol% isoamyl alcohol at a GHSV of 2500 h⁻¹ over 1.0 ml of 1.0 wt% Pt / 0.5 wt% Sn / ZrO₂ catalyst, 3.8% to 98.4% conversion of isoamyl alcohol was achieved at a pressure of 1.0 bar, and at a temperature in the range of 200 to 325 °C. It is also evident that highest conversion (98.4%) was achieved at a temperature of 325 °C. The results of Table 3 are presented graphically in Figure 2.

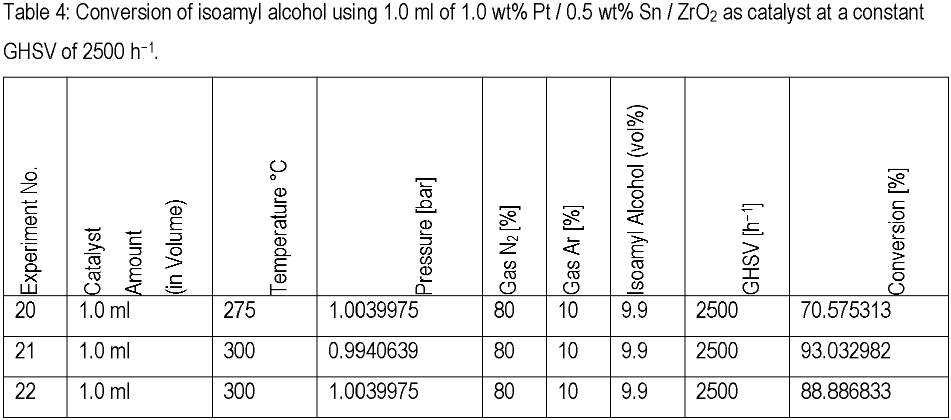

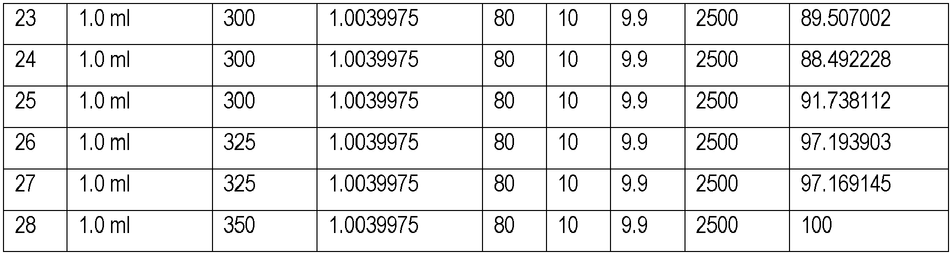

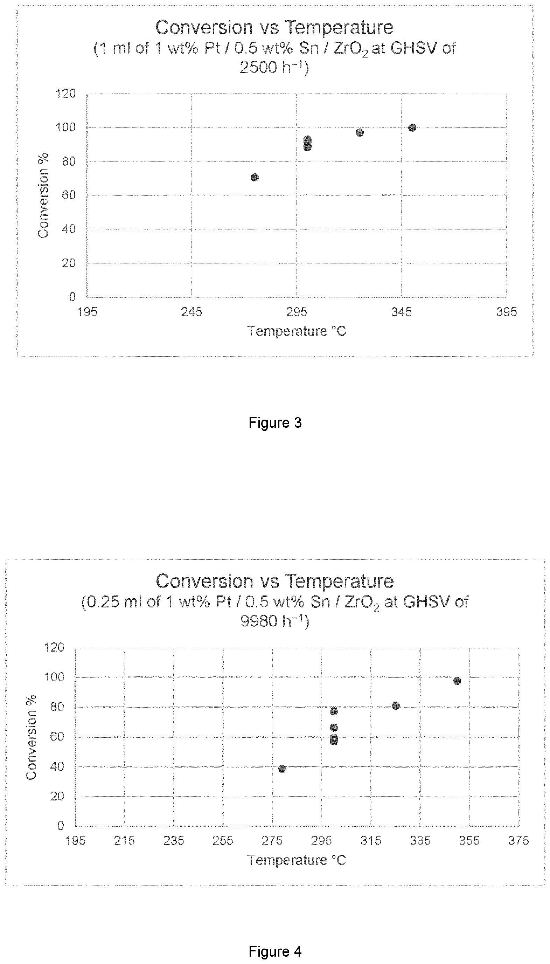

From Table 4, it is evident that on passing a gas feed of 9.9 vol% isoamyl alcohol at a GHSV of 2500 h⁻¹ over 1.0 ml of 1.0 wt% Pt / 0.5 wt% Sn / ZrO₂ catalyst, 70.6% to 100.0% conversion of isoamyl alcohol was achieved at a pressure of 1.0 bar, and at a temperature in the range of 275 to 350 °C. It is also evident that highest conversion (100.0%) was achieved at a temperature of 350 °C. The results of Table 4 are presented graphically in Figure 3. From Experiments 11 to 28, it was observed that the yield of the isobutene was in the range of 1 mol% to 99 mol%.

It is evident from Tables 2-4 that the conversion of Isoamyl alcohol increased with an increase in the amount of the catalyst from 0.25 ml to 1.0 ml, and with a decrease in GHSV of 9.9 vol% isoamyl alcohol from 9970 h⁻¹ to 2500 h⁻¹.

It is evident from Tables 2-4 that the conversion of Isoamyl alcohol in the presence of 1.0 ml of 1.0 wt% Pt / 0.5 wt% Sn / ZrO₂ as catalyst, increased with the temperature in the range of 250 °C to 350 °C. Thus, conversion of Isoamyl alcohol in the presence of the 1.0 wt% Pt / 0.5 wt% Sn / ZrO₂ as catalyst was achieved in the range of 250 °C to 450 °C.

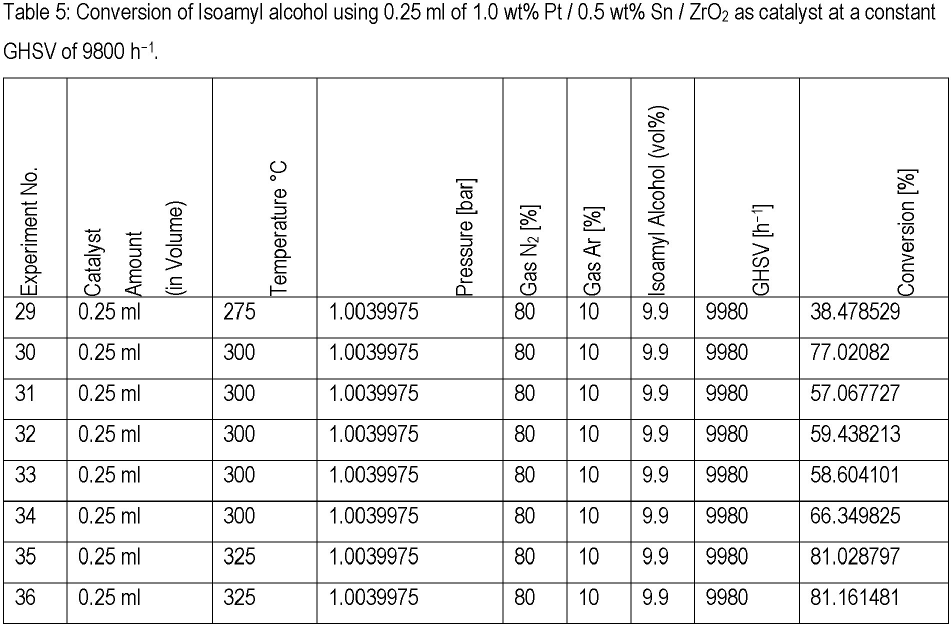

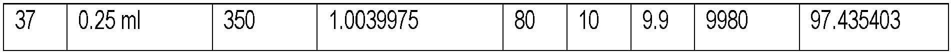

From Table 5, it is evident that on passing a gas feed of 9.9 vol% isoamyl alcohol at a GHSV of 9980 h⁻¹ over 0.25 ml of 1.0 wt% Pt / 0.5 wt% Sn / ZrO₂ catalyst, 38.5% to 97.4% conversion of isoamyl alcohol was achieved at a pressure of 1.0 bar, and at a temperature in the range of 275 to 350 °C. It is also evident that highest conversion (97.4%) was achieved at a temperature of 350 °C. The results of Table 5
are presented graphically in Figure 4.

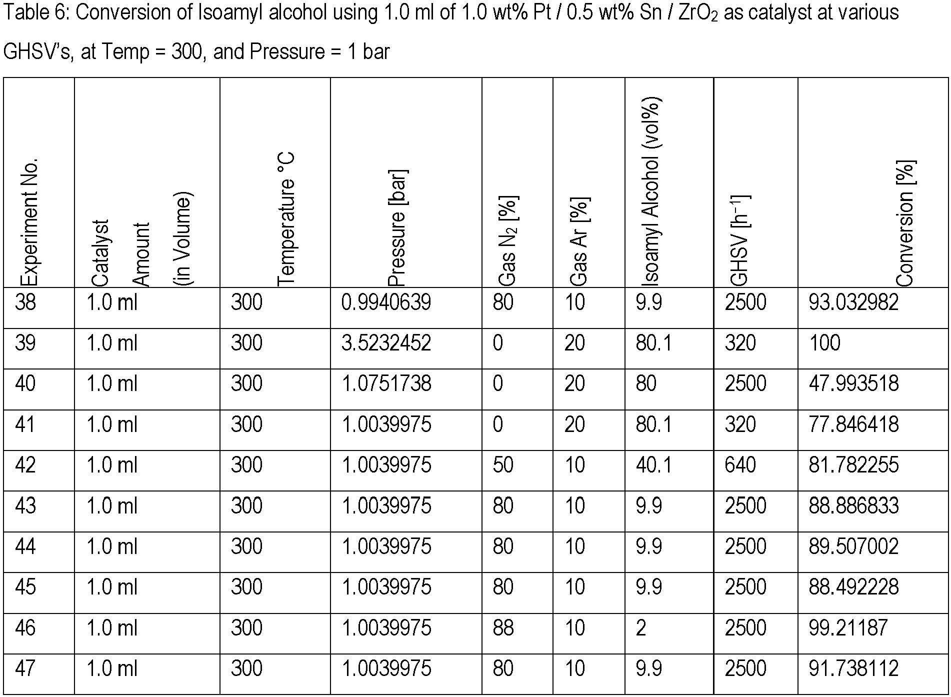

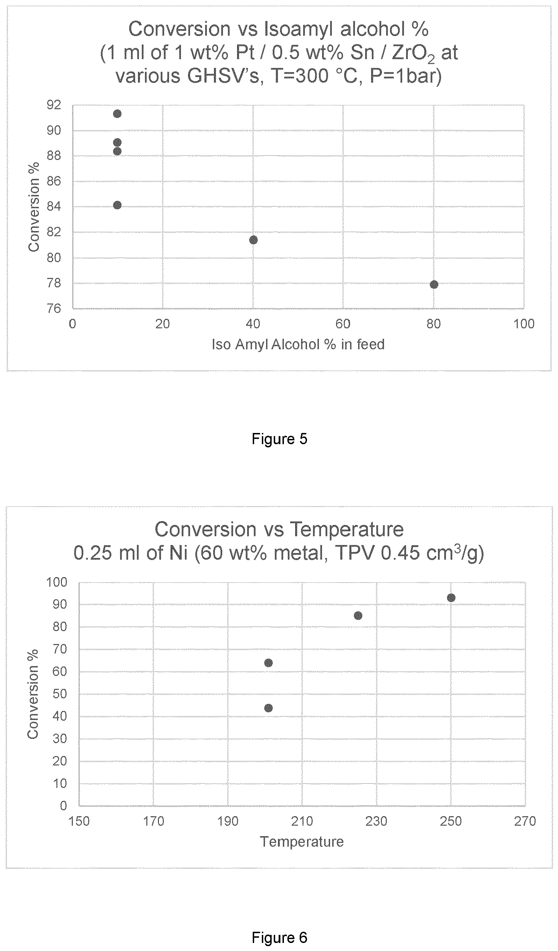

From Table 6, it is evident that on passing a gas feed of 2.0 vol% to 80.1 vol% isoamyl alcohol at a GHSV in the range of 320 h⁻¹ to 2500 h⁻¹, over 0.25 ml of 1.0 wt% Pt / 0.5 wt% Sn / ZrO₂ catalyst, at a pressure of 1.0 bar, and at a temperature of 300 °C, 47.9% to 100.0% conversion of isoamyl alcohol was achieved. The results of Table 6 are presented graphically in Figure 5.

It is evident from Tables 4-6 that the conversion of Isoamyl alcohol decreased with the increasing GHSV. This could be because of the reduction in contact of the isoamyl alcohol in the feed with the catalyst due to increased velocity. Thus, the conversion was optimised by adjusting the amount of isoamyl alcohol in the feed with the change in GHSV in order to increase the contact between isoamyl alcohol in the feed and the catalyst. The conversion of Isoamyl alcohol is highest when the GHSV of the isoamyl alcohol in the feed is in the range of 320 h⁻¹ to 2500 h⁻¹ and the amount of isoamyl alcohol in the feed is in the range of 2.0 vol% to 80.1 vol%.

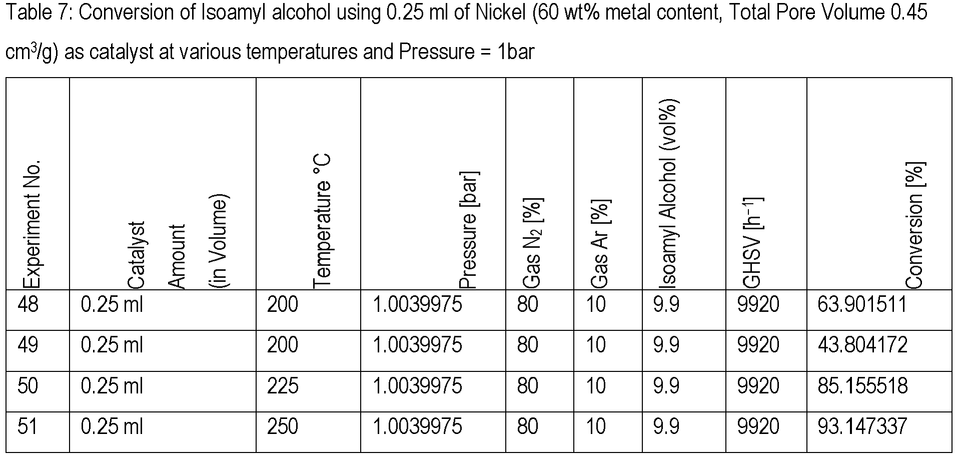

From Table 7, it is evident that on passing a gas feed of 9.9 vol% isoamyl alcohol at a GHSV of 9920 h⁻¹ over 0.25 ml of Nickel (60 wt% metal content, Total Pore Volume 0.45 cm³/g), 43.8% to 93.1 % conversion of isoamyl alcohol was achieved at a pressure of 1.0 bar, and at a temperature in the range of 200 to 250 °C. It is also evident that highest conversion (93.1 %) was achieved at a temperature of 250 °C. The results of Table 7
are presented graphically in Figure 6.

It is evident from Table 7 and Figure 6, that 0.25 ml of Nickel (60 wt% metal content, Total Pore Volume 0.45 cm³/g) as catalyst is actively involved in 43% to 94% conversion of isoamyl alcohol.

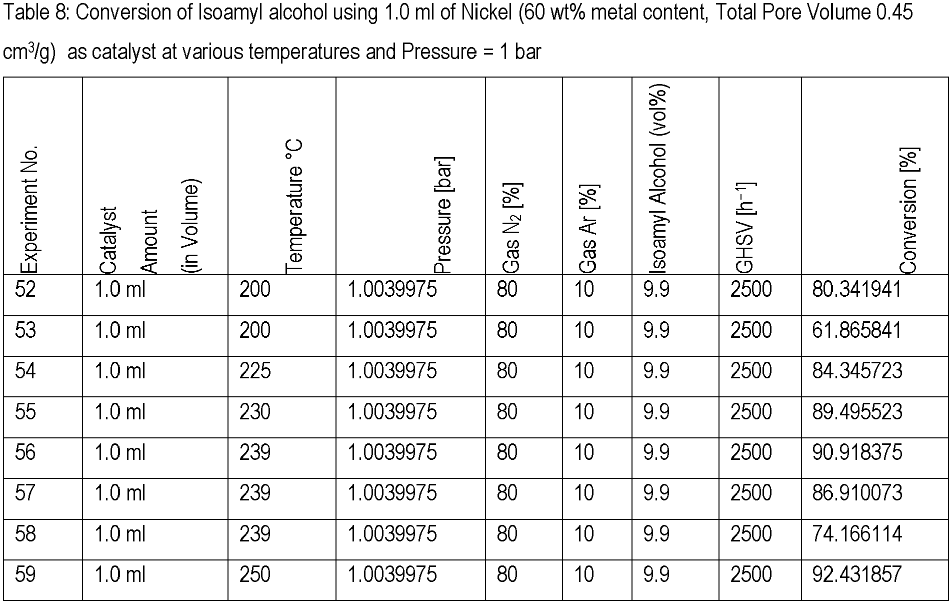

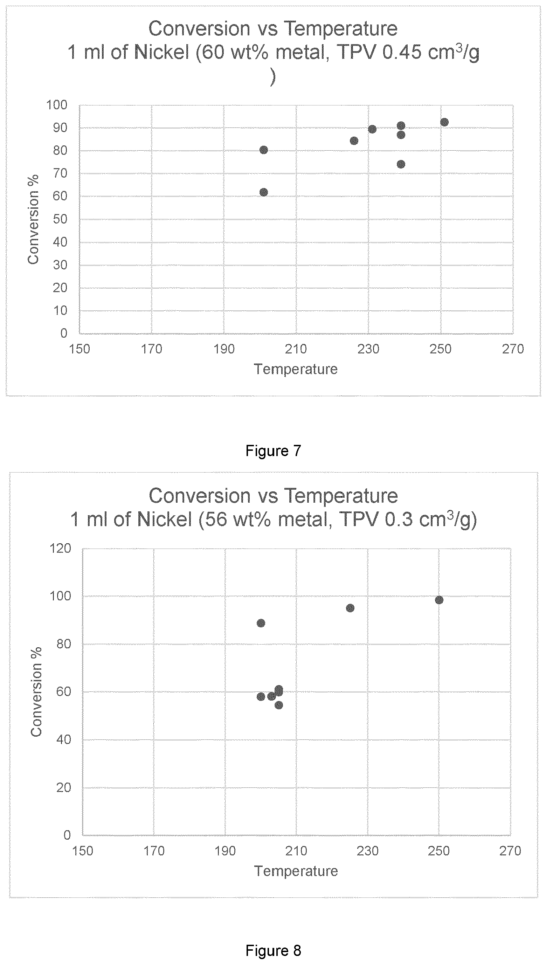

From Table 8, it is evident that on passing a gas feed of 9.9 vol% isoamyl alcohol at a GHSV of 2500 h⁻¹ over 1.0 ml of Nickel (60 wt% metal content, Total Pore Volume 0.45 cm³/g), 61.8% to 92.4% conversion of isoamyl alcohol was achieved at a pressure of 1.0 bar, and at a temperature above 200 °C. It is also evident that highest conversion (92.4%) was achieved at a temperature of 250 °C. The results of Table 8
are presented graphically in Figure 7. From Experiments 52 to 59, it was observed that the yield of the isobutene was in the range of 1 mol% to 99 mol%.

It is evident from Table 8 and Figure 7, that 1.0 ml of Nickel (60 wt% metal content, Total Pore Volume 0.45 cm³/g) as catalyst is actively involved in 61% to 93% conversion of isoamyl alcohol.

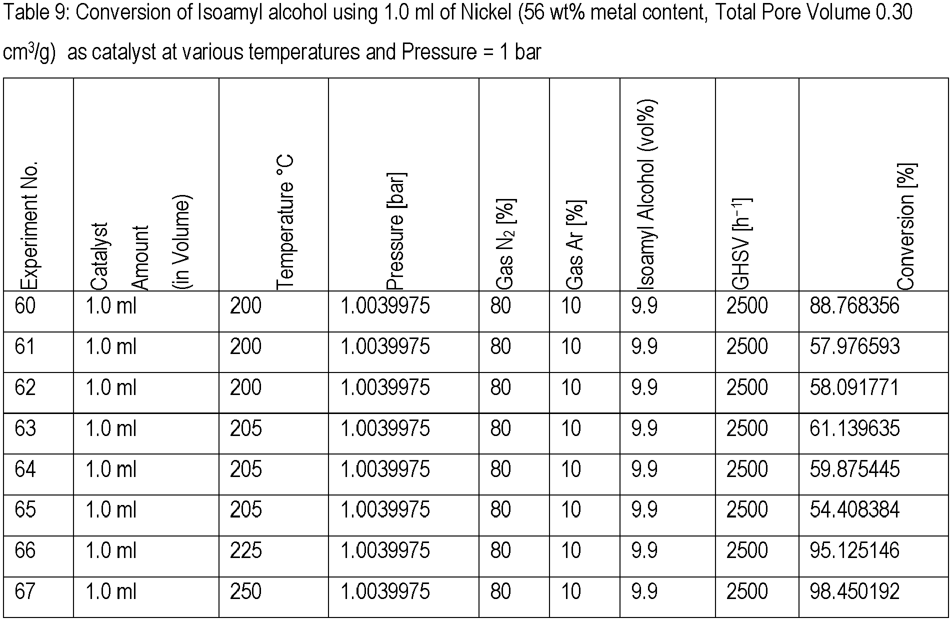

From Table 9, it is evident that on passing a gas feed of 9.9 vol% isoamyl alcohol at a GHSV of 2500 h⁻¹ over 1.0 ml of Nickel (56 wt% metal content, Total Pore Volume 0.3 cm³/g), 54.4% to 98.4% conversion of isoamyl alcohol was achieved at a pressure of 1.0 bar, and at a temperature in the range of 200 to 250 °C. It is also evident that highest conversion (98.4%) was achieved at a temperature of 250 °C. The results of Table 9
are presented graphically in Figure 8. From Experiments 60 to 67, it was observed that the yield of the isobutene was in the range of 1 mol% to 99 mol%.

It is evident from Table 9 and Figure 8, that although 1.0 ml of Ni (56 wt% metal content, Total Pore Volume 0.3 cm³/g) as catalyst is actively involved in 54% to 98.5% conversion of isoamyl alcohol.

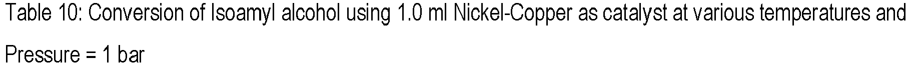

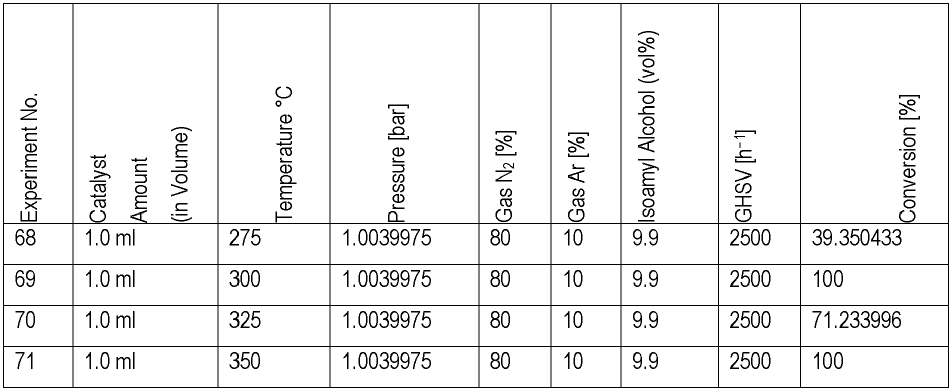

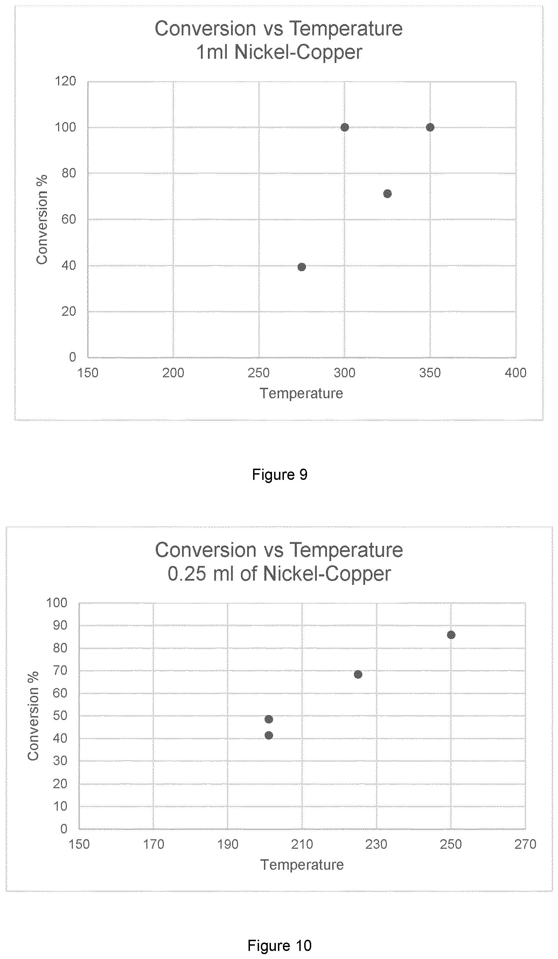

From Table 10, it is evident that on passing a gas feed of 9.9 vol% isoamyl alcohol at a GHSV of 2500 h⁻¹ over 1.0 ml of Nickel-Copper as catalyst, 39.3% to 100.0% conversion of isoamyl alcohol was achieved at a pressure of 1.0 bar, and at a temperature in the range of 275 to 350 °C. It is also evident that highest conversion (100.0%) was achieved at a temperature above 300 °C. The results of Table 10 are presented graphically in Figure 9. From Experiments 68 to 71, it was observed that the yield of the isobutene was in the range of 1 mol% to 99 mol%.

It is evident from Table 10 and Figure 9, that Nickel-Copper as catalyst is actively involved in 39% to 100% conversion of isoamyl alcohol.

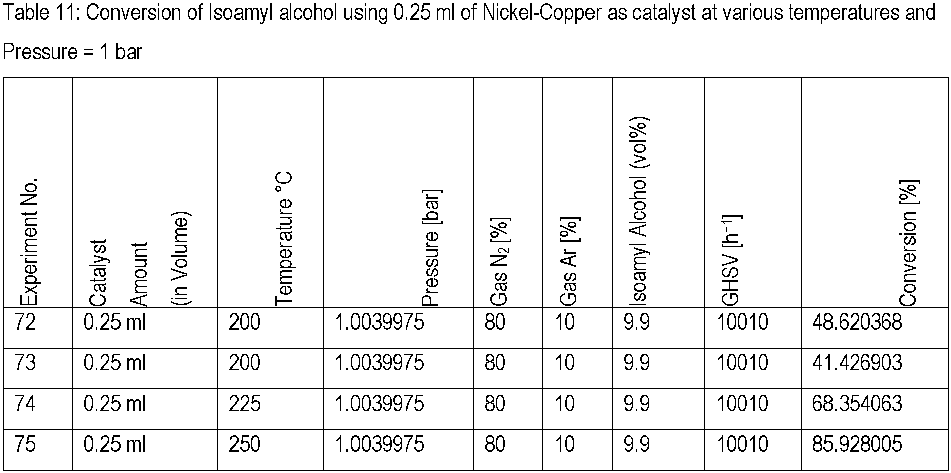

From Table 11, it is evident that on passing a gas feed of 9.9 vol% isoamyl alcohol at a GHSV of 10010 h⁻¹ over 0.25 ml of Nickel-Copper as catalyst, 41.4% to 85.9% conversion of isoamyl alcohol was achieved at a pressure of 1.0 bar, and at a temperature in the range of 200 to 250 °C. It is also evident that highest conversion (85.9%) was achieved at temperature 250 °C. The results of Table 11 are presented graphically in Figure 10.

It is evident from Table 11 and Figure 10 that 0.25 ml of Nickel-Copper as catalyst is actively involved in 41% to 86% conversion of isoamyl alcohol.

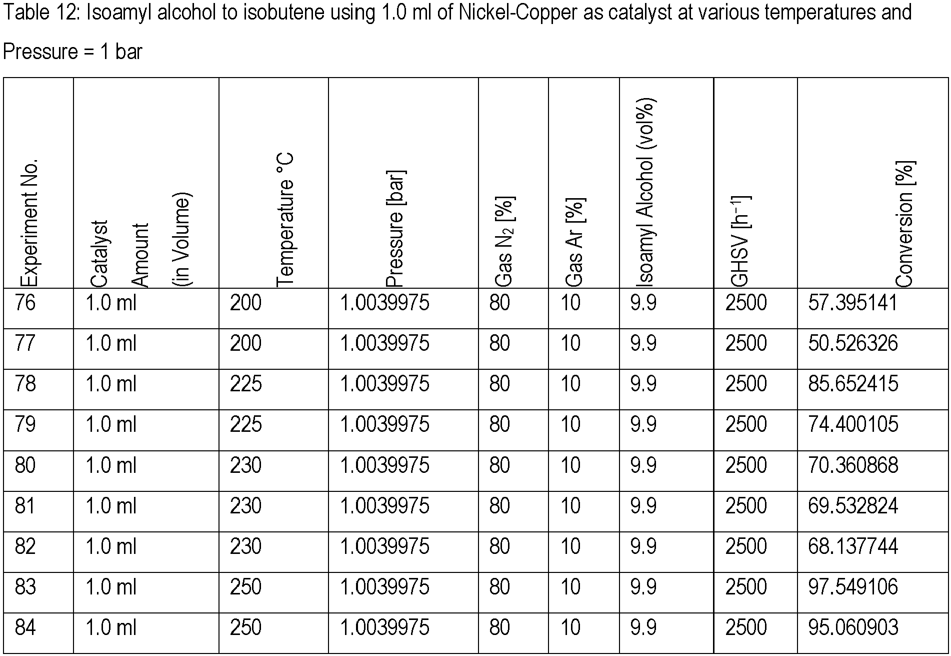

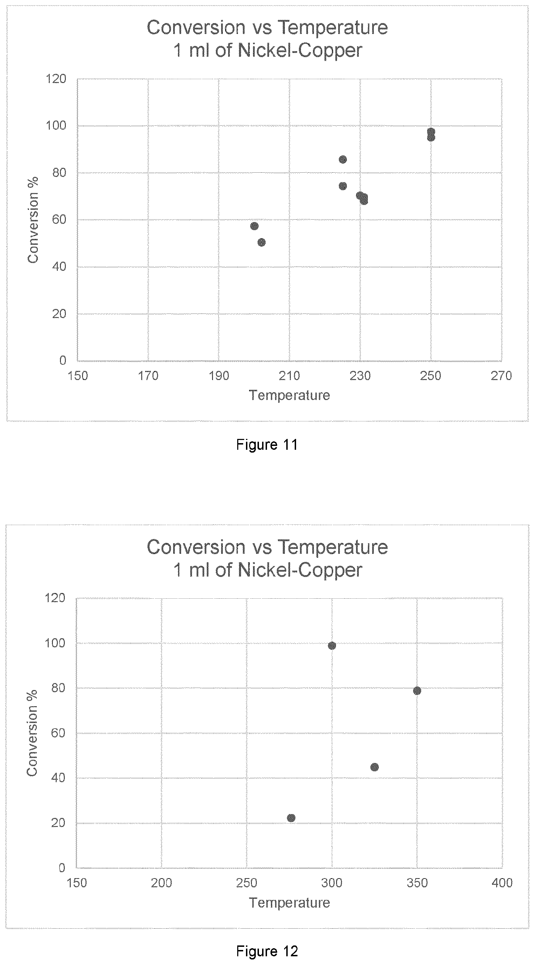

From Table 12, it is evident that on passing a gas feed of 9.9 vol% isoamyl alcohol at a GHSV of 2500 h⁻¹ over 1.0 ml of Nickel-Copper as catalyst, 50.5% to 97.5% conversion of isoamyl alcohol was achieved at a pressure of 1.0 bar, and at a temperature in the range of 200 to 250 °C. It is also evident that highest conversion (97.5%) was achieved at temperature 250 °C. The results of Table 12 are presented graphically in Figure 11. From Experiments 76 to 84, it was observed that the yield of the isobutene was in the range of 1 mol% to 99 mol%.

It is evident from Table 12 and Figure 11, that 1.0 ml of Nickel-Copper as catalyst is actively involved in 50% to 98% conversion of isoamyl alcohol.

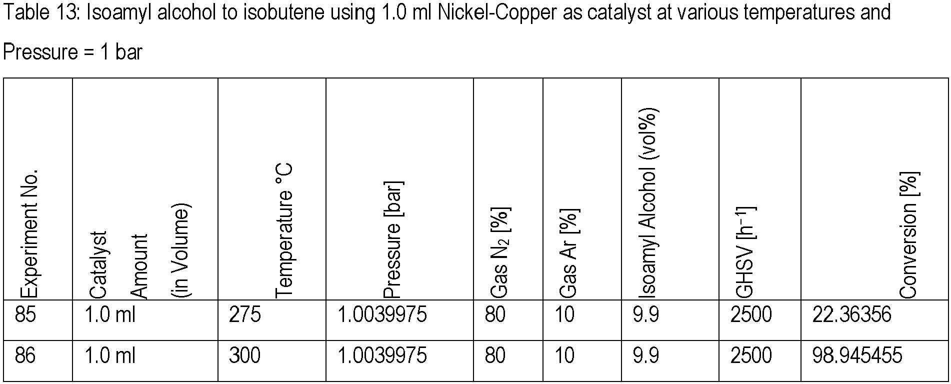

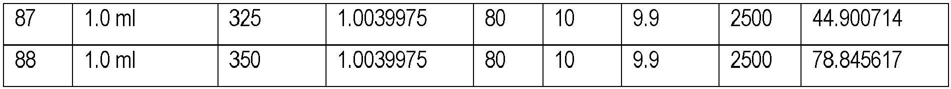

From Table 13, it is evident that on passing a gas feed of 9.9 vol% isoamyl alcohol at a GHSV of 2500 h⁻¹ over 1.0 ml of Nickel-Copper as catalyst, 22.3% to 98.9% conversion of isoamyl alcohol was achieved at a pressure of 1.0 bar, and at a temperature in the range of 275 to 350 °C. It is also evident that highest conversion (98.9%) was achieved at temperature 300 °C. The results of Table 13 are presented graphically in Figure 12. From Experiments 85 to 88, it was observed that the yield of the isobutene was in the range of 1 mol% to 99 mol%.

It is evident from Table 13 and Figure 12 that Nickel-Copper as catalyst is actively involved in 22% to 99% conversion of isoamyl alcohol.

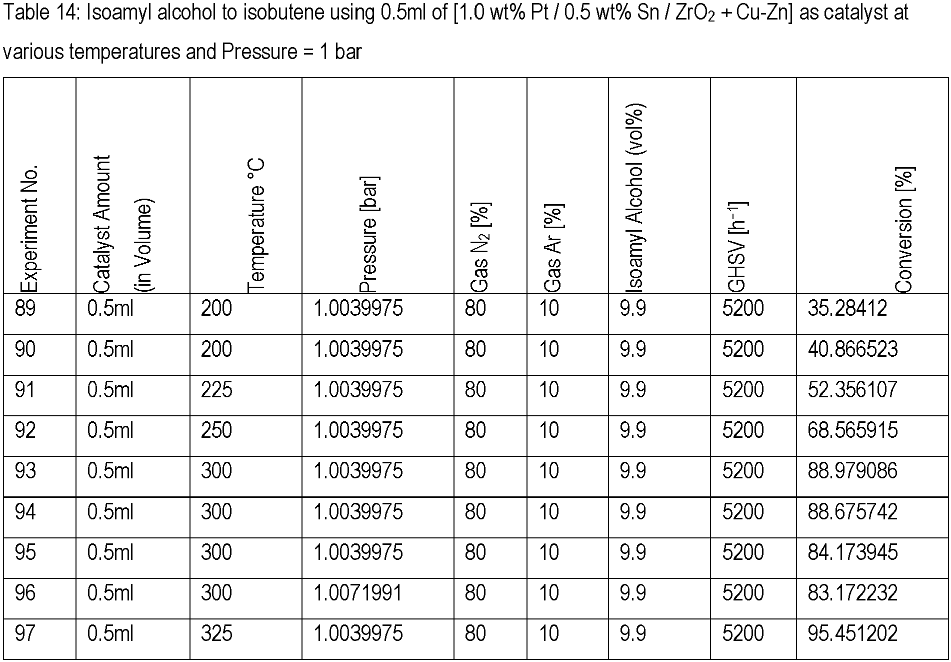

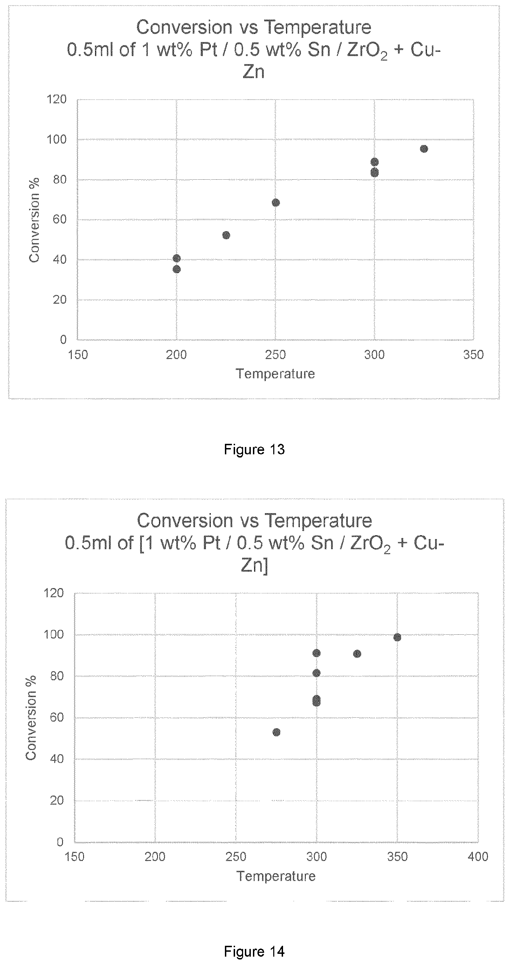

From Table 14, it is evident that on passing a gas feed of 9.9 vol% isoamyl alcohol at a GHSV of 5200 h⁻¹ over 0.5ml of [1.0 wt% Pt / 0.5 wt% Sn / ZrO₂ + Cu-Zn] as catalyst, 35.3% to 95.4% conversion of isoamyl alcohol was achieved at a pressure of 1.0 bar, and at a temperature in the range of 200 to 325 °C. It is also evident that highest conversion (95.4%) was achieved at temperature 325 °C. The results of Table 14
are presented graphically in Figure 13.

It is evident from Table 14 and Figure 13, that 0.5 ml of [1.0 wt% Pt / 0.5 wt% Sn / ZrO₂ + Cu-Zn] as catalyst is actively involved in 35% to 96% conversion of isoamyl alcohol.

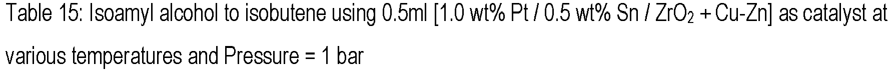

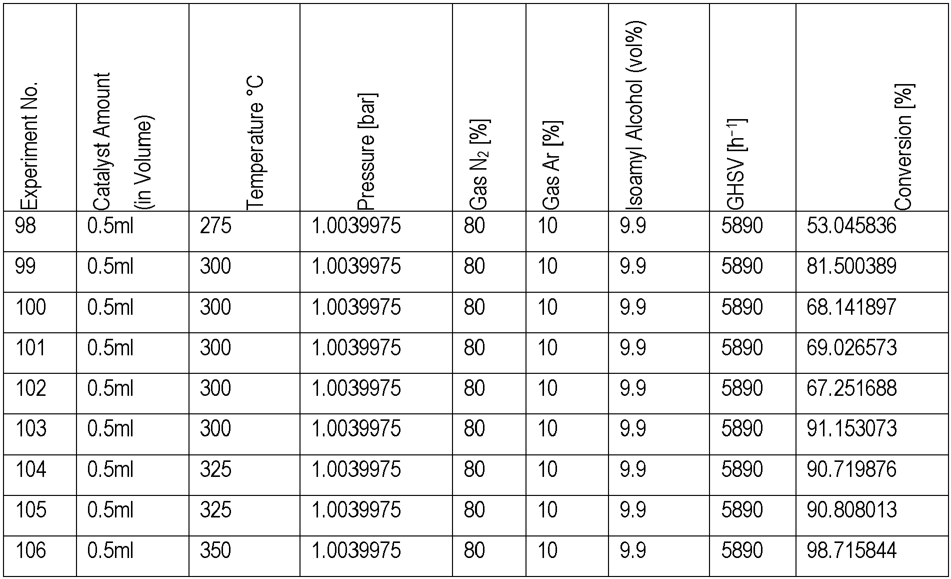

From Table 15, it is evident that on passing a gas feed of 9.9 vol% isoamyl alcohol at a GHSV of 5890 h⁻¹ over 0.5ml of [1.0 wt% Pt / 0.5 wt% Sn / ZrO₂ + Cu-Zn] as catalyst, 53.0% to 98.7% conversion of isoamyl alcohol was achieved at a pressure of 1.0 bar, and at a temperature in the range of 275 to 350 °C. It is also evident that highest conversion (98.7%) was achieved at temperature 350 °C. The results of Table 15
are presented graphically in Figure 14.

It is evident from Table 15 and Figure 14 that 1.0 wt% Pt / 0.5 wt% Sn / ZrO₂ + Cu-Zn as catalyst is actively involved in 53% to 99% conversion of isoamyl alcohol.

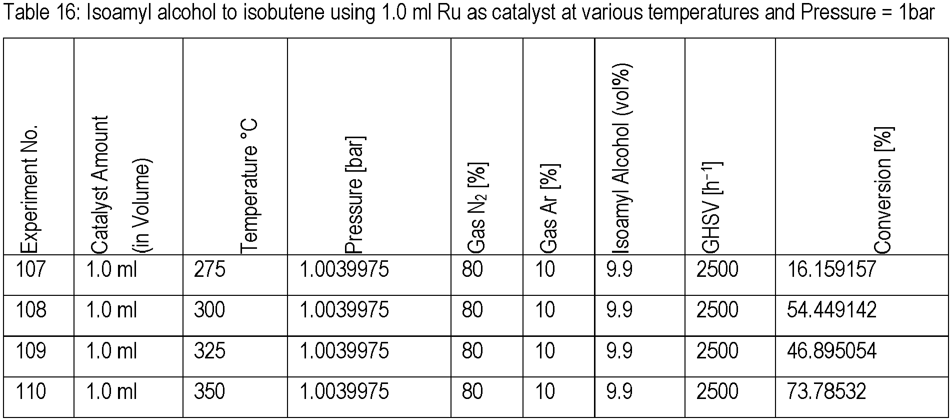

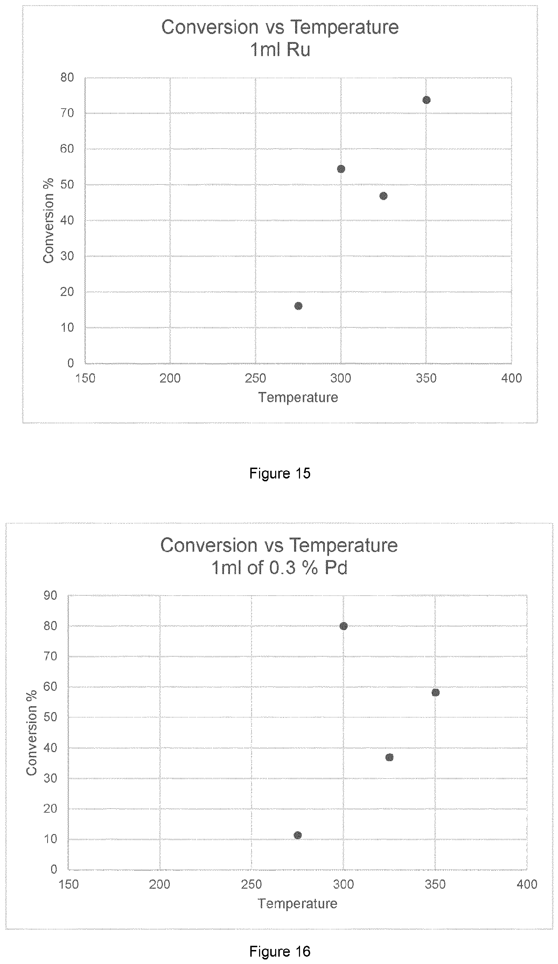

From Table 16, it is evident that on passing a gas feed of 9.9 vol% isoamyl alcohol at a GHSV of 2500 h⁻¹ over 1.0 ml of Ru as catalyst, 16.1% to 73.8% conversion of isoamyl alcohol was achieved at a pressure of 1.0 bar, and at a temperature in the range of 275 to 350 °C. It is also evident that highest conversion (93.8%) was achieved at temperature 350 °C. The results of Table 16 are presented graphically in Figure 15.

It is evident from Table 16 and Figure 15, that 1.0 ml Ru as catalyst is actively involved in 16% to 74% conversion of isoamyl alcohol.

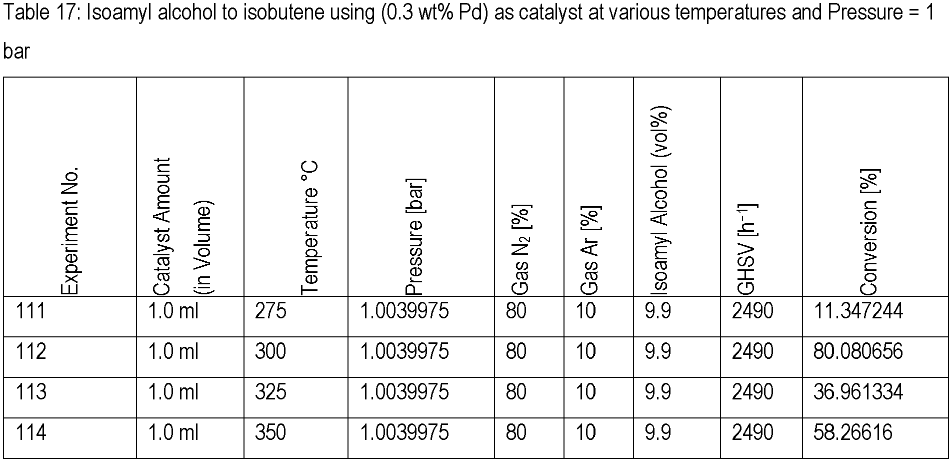

From Table 17, it is evident that on passing a gas feed of 9.9 vol% isoamyl alcohol at a GHSV of 2490 h⁻¹ over 1.0 ml of 0.3 % Pd as catalyst, 11.3% to 80.0% conversion of isoamyl alcohol was achieved at a pressure of 1.0 bar, and at a temperature in the range of 275 to 350 °C. It is also evident that highest conversion (80.0%) was achieved at temperature 300 °C. The results of Table 20 are presented graphically in Figure 16.

It is evident from Table 20 and Figure 16 that (0.3 % Pd) as catalyst is actively involved in 11% to 81% conversion of isoamyl alcohol.

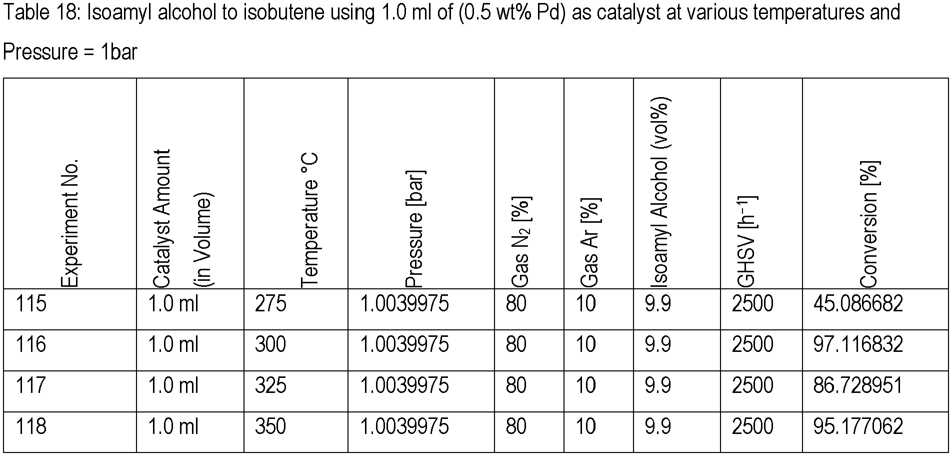

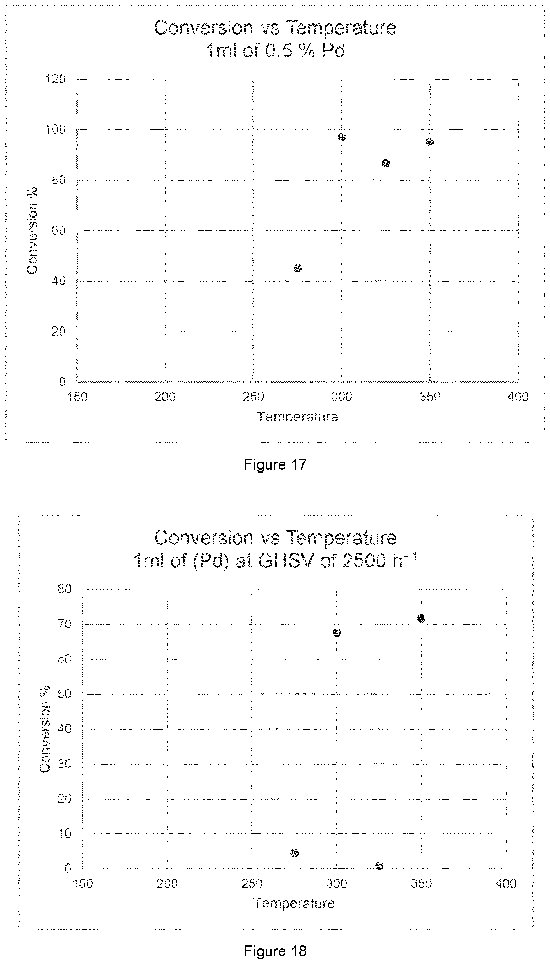

From Table 18, it is evident that on passing a gas feed of 9.9 vol% isoamyl alcohol at a GHSV of 2500 h⁻¹ over 1.0 ml of 0.5 % Pd as catalyst, 45.1% to 97.1% conversion of isoamyl alcohol was achieved at a pressure of 1.0 bar, and at a temperature in the range of 275 to 350 °C. It is also evident that highest conversion (97.1%) was achieved at temperature 300 °C. The results of Table 18 are presented graphically in Figure17.

It is evident from Table 18 and Figure 17, that (0.5 % Pd) as catalyst is actively involved in 45% to 97% conversion of isoamyl alcohol.

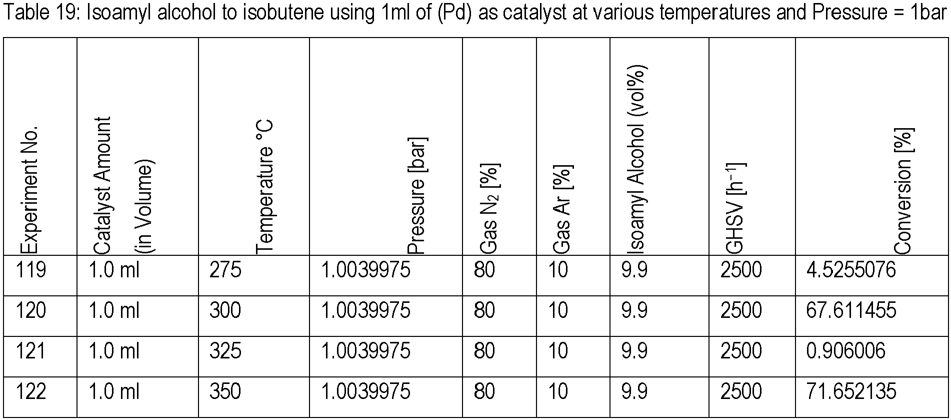

From Table 19, it is evident that on passing a gas feed of 9.9 vol% isoamyl alcohol at a GHSV of 2500 h⁻¹ over 1.0 ml of (Pd) as catalyst, 0.9% to 71.6% conversion of isoamyl alcohol was achieved at a pressure of 1.0 bar, and at a temperature in the range of 275 to 350 °C. The results of Table 19 are presented graphically in Figure 18. From Experiments 119 to 122, it was observed that the yield of the isobutene was in the range of 1 mol% to 99 mol%.

It is evident from Table 19 and Figure 18 that (Pd) as catalyst is actively involved in 0.9% to 71% conversion of isoamyl alcohol.

## Claims

1. A process for preparing polyisobutene by polymerising an isobutene-containing olefin mixture in the presence of at least one Lewis Acid and in the presence of at least one initiator, **characterised in that** the isobutene in the olefin mixture is at least partly obtained by a process for preparing mixtures of lower olefins with 4 to 7 carbon atoms, comprising the steps of:
a. feeding into a reaction vessel a stream comprising linear and/or branched C₅-C₈ primary alcohol which has at least one hydrogen atom attached to the carbon atom adjacent to the one bearing the alcohol group,
b. providing a catalyst comprising at least one catalytically active metal selected from platinum (Pt), palladium (Pd), nickel (Ni), Chromium (Cr), Molybdenum (Mo), Ruthenium (Ru), or combinations thereof, and
c. subjecting the primary alcohol to a de-hydroformulation in the presence of the catalyst of step b) yielding an olefin having one carbon atom less than the starting alcohol,
wherein the linear and/or branched C₅-C₈ primary alcohol comprises 3-Methyl 1-butanol.

2. A process for preparing polyisobutene from a mixture of lower olefins with 4 to 7 carbon atoms, comprising the steps of:
a) feeding into a reaction vessel a stream comprising linear and/or branched C₅-C₈ primary alcohol which has at least one hydrogen atom attached to the carbon atom adjacent to the one bearing the alcohol group,
b) providing a catalyst comprising at least one catalytically active metal selected from platinum (Pt), palladium (Pd), nickel (Ni), Chromium (Cr), Molybdenum (Mo), Ruthenium (Ru), or combinations thereof,
c) subjecting the primary alcohol to a de-hydroformulation in the presence of the catalyst of step b) yielding an olefin having one carbon atom less than the starting alcohol,
d) optionally purifying the reaction product from step c) yielding an olefin-containing product stream, and
e) polymerising the olefin obtained in step c) or d) (if applicable) optionally together with olefin from other sources in the presence of at least one Lewis Acid and in the presence of at least one initiator,
wherein the linear and/or branched C₅-C₈ primary alcohol comprises 3-Methyl 1-butanol and the mixture of olefins comprises isobutene.

3. The process according to claim 1or 2, wherein the stream comprising linear and/or branched C₅- C₈ primary alcohol which has at least one hydrogen atom attached to the carbon atom adjacent to the one bearing the alcohol group has a pMC greater than 90, when measured by a method as described in the ASTM norm D6866.

4. The process according to claim 3, wherein the stream comprising linear and/or branched C₅- C₈ primary alcohol which has at least one hydrogen atom attached to the carbon atom adjacent to the one bearing the alcohol group is obtained from natural sources or from a fermentation process.

5. The process according to any of the claims 1 to 4, wherein the stream comprising linear and/or branched C₅-C₈ primary alcohol which has at least one hydrogen atom attached to the carbon atom adjacent to the one bearing the alcohol group is converted to at least one lower olefin having C₄-C₇ carbon atoms at a yield of at least about 80 wt.% of the maximum theoretical molar yield.

6. The process according to any of the claims 1 to 4, wherein the process comprises the steps of:
a) feeding into a reaction vessel a stream comprising linear and/ or branched C₅-C₈ primary alcohol which has at least one hydrogen atom attached to the carbon atom adjacent to the one bearing the alcohol group,
b) providing a catalyst comprising at least one catalytically active metal selected from platinum (Pt), palladium (Pd), nickel (Ni), Chromium (Cr), Molybdenum (Mo), Ruthenium (Ru), or combinations thereof, and
c) subjecting the primary alcohol to a de-hydroformulation in the presence of the catalyst of step b) yielding an olefin having one carbon atom less than the starting alcohol,
wherein step c) is conducted at a partial pressure in the range of 0.02 to 4.0 bar, and at a temperature in the range of 200 °C to 450 °C, preferably 250 °C to 450 °C, more preferably 300 to 450 °C, even more preferably 300 °C to 400 °C, especially 300 °C to 350 °C.

7. The process according to any of the preceding claims, wherein the Lewis Acid is selected from the group consisting of aluminium trihalide, alkylaluminium halide, iron trihalide, a gallium trihalide, a titanium tetrahalide, a zinc dihalide, a tin dihalide, a tin tetrahalide, and a boron trihalide, more preferably selected from the group consisting of aluminium trihalide, alkylaluminium halide, titanium tetrahalide, and boron trihalide, even more preferably selected from the group consisting of aluminium trihalide, alkylaluminium halide, and boron trihalide, and especially the metal halide is aluminium trihalide or boron trihalide or even aluminium trichloride or boron trifluoride.

8. The process according to any of the preceding claims, wherein the Lewis Acid forms a complex with a donor, comprising at least one oxygen and/or nitrogen atom with at least one lone electron pair, preferably at least one oxygen atom with at least one lone electron pair and very preferably are selected from the group consisting of organic compounds with at least one ether function, organic compounds with at least one carboxylic ester function, organic compounds with at least one aldehyde function, organic compounds with at least one keto function, and organic compounds with at least one nitrogen containing heterocyclic ring.

9. The process according to any of the preceding claims, wherein the initiator is selected from organic hydroxyl compounds, organic halogen compounds and water.

10. Use of an isobutene-containing monomer mixture obtained from a process comprising the steps a) to c) as described in claims 1 to 6 in a polymerisation process.

11. Polyisobutene obtainable by a process according to any of the preceding claims.

12. Polyisobutene according to claim 11 predominantly using olefin obtained from step c) or d) (if applicable) which exhibits a pMC greater than 90, when measured by a method as described in the ASTM norm D6866.

13. Polyisobutene according to claim 11 exhibiting a pMC of at least 1%, preferably at least 2%, more preferably at least 5%, even more preferably at least 10%, and especially at least 25%, when measured by a method as described in the ASTM norm D6866.

14. Polyisobutene according to Claim 11 exhibiting a pMC of at least 90%, preferably at least 95%, more preferably at least 98%, even more preferably at least 99%, or even 100%, when measured by a method as described in the ASTM norm D6866.

15. Use of polyisobutene according to claim 14 in food contact- or medical applications, preferably for chewing gums, in adhesive compositions approved for food contact or in plasters.
